# EUROPEAN PATENT APPLICATION

(11) **EP 1 626 057 A1**
(43) Date of publication of application: **15.02.2006**
(21) Application number: 05016083.7
(22) Date of filing: 25.07.2005
(51) Int. Cl.: C07K 14/47, C07K 14/72

(54) **Chimpanzee trace amine associated receptors**

(30) Priority: 30.07.2004 EP 04103673
(71) Applicant: F.HOFFMANN-LA ROCHE AG, 4070 Basel (CH)
(72) Inventor: Ebeling, Martin, 79639 Grenzach-Wyhlen (DE); Hoener, Marius, 4056 Basel (CH); Lindemann, Lothar, 4057 Basel (CH)

(57) **Abstract**

The present invention provides a fingerprint sequence which is specific and selective for chimp trace amine associated receptors (TAAR) forming a subfamily of G protein coupled receptors. The invention also provides the novel chimp polypeptides identified as members of this family, nucleic acids encoding said polypeptides, and vectors and host cells comprising the novel family members. In addition, the invention provides methods of identifying chimp TAARs.

SEQUENCE LISTING

<110>  F. Hoffmann-La Roche

<120>  Chimpanzee Trace Amine Associated Receptors

<130>  21677

<160>  40

<170>  PatentIn version 3.2

<210>  1
<211>  16
<212>  PRT
<213>  Mus musculus


<220>
<221>  fingerprint
<222>  (1)..(16)

<220>
<221>  misc_feature
<222>  (3)..(4)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(12)
<223>  Xaa can be any naturally occurring amino acid

<220>
<221>  misc_feature
<222>  (14)..(14)
<223>  Xaa can be any naturally occurring amino acid

<400>  1

Asn Ser Xaa Xaa Asn Pro Xaa Xaa Tyr Xaa Xaa Xaa Tyr Xaa Trp Phe
1               5                   10                  15


<210>  2
<211>  1020
<212>  DNA
<213>  Pan troglodytes


<220>
<221>  ptTAAR1
<222>  (1)..(1020)

<400>  2
atgatgccct tttgccacaa tataattaat atttcctgtg tgaaaaacaa ctggtcaaat      60

gatgtccgtg cttccctgta cagtttaatg gtgctcataa ttctgaccac actcgttggc     120

aatctgatag ttattgtttc tatatcacac ttcaaagaac ttcatacccc gacaaattgg     180

ctcattcatt ccatggccac tgtggacttt cttccggggt gtctggtcat gccttacagt     240

atggtgagat ctgctgagca ttgttggtat tttggagaag tcttctgtaa aattcacaca     300

KM/20.05.2005

agcaccgaca ttatgctgag ctcagcctcc attttccatt tgtctttcat ctccattgac 360

cgctactatg ctgtgtgtga tccactgaga tataaagcca agatcaatat cttggttatt 420

tgtgtgatga tcttcattag ttggagtgtc cccgctgttt ttgcatttgg aatgatcttt 480

ctggagctaa acttcaaagg cgctgaagag atatattaca aacatgttca ctgcagagga 540

ggttgctctg tcttctttag caaaatatct ggggtactga cctttatgac ttctttttat 600

atacctggat ctattatgtt atgtgtctat tacagaatat atcttatcgc taaagagcag 660

gcaagattaa ttaatgatgc caatcagaag ctccaaattg gattggaaat gaaaaatgga 720

atttcacaaa gcaaagaaag gaaagctgtg aagacattgg ggattgtgat gggagttttc 780

ctaatatgct ggtgcccttt ctttatctgt acagtcatgg acccttttct tcactacatt 840

attccaccta ctttgaatga tgtattgatt tggtttggct acttgaactc tacatttaat 900

ccaatggttt atgcattttt ctatccttgg tttagaaaag cactgaagat gatgctgttt 960

ggtaaaattt tccaaaaaga ttcatccagg tgtaaattat ttttggaatt gagttcatag 1020

<210> 3
<211> 339
<212> PRT
<213> Pan troglodytes

<220>
<221> ptTAAR1
<222> (1)..(339)

<400> 3

Met Met Pro Phe Cys His Asn Ile Ile Asn Ile Ser Cys Val Lys Asn
1               5                   10                  15

Asn Trp Ser Asn Asp Val Arg Ala Ser Leu Tyr Ser Leu Met Val Leu
            20                  25                  30

Ile Ile Leu Thr Thr Leu Val Gly Asn Leu Ile Val Ile Val Ser Ile
        35                  40                  45

Ser His Phe Lys Glu Leu His Thr Pro Thr Asn Trp Leu Ile His Ser
    50                  55                  60

Met Ala Thr Val Asp Phe Leu Pro Gly Cys Leu Val Met Pro Tyr Ser
65                  70                  75                  80

Met Val Arg Ser Ala Glu His Cys Trp Tyr Phe Gly Glu Val Phe Cys
                85                  90                  95

Lys Ile His Thr Ser Thr Asp Ile Met Leu Ser Ser Ala Ser Ile Phe
            100                 105                 110

His Leu Ser Phe Ile Ser Ile Asp Arg Tyr Tyr Ala Val Cys Asp Pro
        115                 120                 125

Leu Arg Tyr Lys Ala Lys Ile Asn Ile Leu Val Ile Cys Val Met Ile
    130                 135                 140

Phe Ile Ser Trp Ser Val Pro Ala Val Phe Ala Phe Gly Met Ile Phe
145                 150                 155                 160

Leu Glu Leu Asn Phe Lys Gly Ala Glu Glu Ile Tyr Tyr Lys His Val
                165                 170                 175

His Cys Arg Gly Gly Cys Ser Val Phe Phe Ser Lys Ile Ser Gly Val
            180                 185                 190

Leu Thr Phe Met Thr Ser Phe Tyr Ile Pro Gly Ser Ile Met Leu Cys
        195                 200                 205

Val Tyr Tyr Arg Ile Tyr Leu Ile Ala Lys Glu Gln Ala Arg Leu Ile
    210                 215                 220

Asn Asp Ala Asn Gln Lys Leu Gln Ile Gly Leu Glu Met Lys Asn Gly
225                 230                 235                 240

Ile Ser Gln Ser Lys Glu Arg Lys Ala Val Lys Thr Leu Gly Ile Val
                245                 250                 255

Met Gly Val Phe Leu Ile Cys Trp Cys Pro Phe Phe Ile Cys Thr Val
            260                 265                 270

Met Asp Pro Phe Leu His Tyr Ile Ile Pro Pro Thr Leu Asn Asp Val
        275                 280                 285

Leu Ile Trp Phe Gly Tyr Leu Asn Ser Thr Phe Asn Pro Met Val Tyr
    290                 295                 300

Ala Phe Phe Tyr Pro Trp Phe Arg Lys Ala Leu Lys Met Met Leu Phe
305                 310                 315                 320

Gly Lys Ile Phe Gln Lys Asp Ser Ser Arg Cys Lys Leu Phe Leu Glu
                325                 330                 335

Leu Ser Ser

<210>  4
<211>  1055
<212>  DNA
<213>  Pan troglodytes

<220>
<221>  ptTAAR2 Y
<222>  (1)..(1055)
<223>  pseudogene

<400>  4
atggctgtct catcagagca agatgaactt tcacatttca aaagaacaca gacaaaaaag      60

gaaacattca attgctctga atatggaaat agatcttgcc cagaaaatga aagatctctg 120

ggtgtccgag tggctatgta ttcatttatg gcgggatcca tattcatcac aatatttggc 180

aatcttgccg tgataatttc catttcctac ttcaagcagc ttcacacacc aaccaacttc 240

ctcatcctct ccatggccat cactgatttc ctcctgggat tcaccatcat gccatatagt 300

atgatcagat cagtggagaa ctgctggtat tttgggctta cattttgcaa gattcattat 360

agttttgatc tgatgcttag cataacatcc atttttcatc tttgctcagt ggccattgat 420

agattttatg ctgtctgtta cccattacgt tattccacca aaataactat tccagtcatt 480

aaaagattgc tacttctatg ttggtcggtc cctggagcat ttgccttcgg ggtggtcttc 540

tcagaggcct atgcagatgg aatagagggc tatgacatct tggttgcttg ttccagttcc 600

tgcccagtga tgttcaacaa gctatggggg accaccttgt ttatggcagg tttcttcact 660

cctgggtcta tgatggtggg gatttatggc aaaatttttg cggtatccag aaaacatgct 720

catgccatca ataacttgca agaaaatcaa aataatcaag tgaagaaaga caaaaaagct 780

gccaaaactt taggaatagt gataggagtt ttcttattat gttggtttcc ttctttcttc 840

acaattttat tggatccctt ttgaacttct ctactcctgt agttttgttt gatgccttga 900

catggtttgg ctattttaac tccacatgta atccgttaat atatggtttc ttctatccct 960

ggtttcgcag agcactgaag tacattttgc taggtaaaat tttcagctca tgtttccata 1020

ctactaattt gtgtatgcaa aaagagagtg agtag 1055

<210> 5
<211> 1056
<212> DNA
<213> Pan troglodytes

<220>
<221> ptTAAR2 Y f
<222> (1)..(1056)
<223> fixed pseudogene

<400> 5
atggctgtct catcagagca agatgaactt tcacatttca aaagaacaca gacaaaaaag 60

gaaacattca attgctctga atatggaaat agatcttgcc cagaaaatga aagatctctg 120

ggtgtccgag tggctatgta ttcatttatg gcgggatcca tattcatcac aatatttggc 180

aatcttgccg tgataatttc catttcctac ttcaagcagc ttcacacacc aaccaacttc 240

ctcatcctct ccatggccat cactgatttc ctcctgggat tcaccatcat gccatatagt 300

atgatcagat cagtggagaa ctgctggtat tttgggctta cattttgcaa gattcattat 360

agttttgatc tgatgcttag cataacatcc atttttcatc tttgctcagt ggccattgat 420

agattttatg ctgtctgtta cccattacgt tattccacca aaataactat tccagtcatt 480

aaaagattgc tacttctatg ttggtcggtc cctggagcat ttgccttcgg ggtggtcttc 540

tcagaggcct atgcagatgg aatagagggc tatgacatct tggttgcttg ttccagttcc 600

tgcccagtga tgttcaacaa gctatggggg accaccttgt ttatggcagg tttcttcact 660

cctgggtcta tgatggtggg gatttatggc aaaatttttg cggtatccag aaaacatgct 720

catgccatca ataacttgca agaaaatcaa aataatcaag tgaagaaaga caaaaaagct 780

gccaaaactt taggaatagt gataggagtt ttcttattat gttggtttcc ttctttcttc 840

acaattttat tggatccctt tttgaacttc tctactcctg tagttttgtt tgatgccttg 900

acatggtttg gctattttaa ctccacatgt aatccgttaa tatatggttt cttctatccc 960

tggtttcgca gagcactgaa gtacattttg ctaggtaaaa ttttcagctc atgtttccat 1020

actactaatt tgtgtatgca aaaagagagt gagtag 1056

<210> 6
<211> 351
<212> PRT
<213> Pan troglodytes

<220>
<221> ptTAAR2 Yf
<222> (1)..(351)
<223> protein of fixed pseudogene

<400> 6

Met Ala Val Ser Ser Glu Gln Asp Glu Leu Ser His Phe Lys Arg Thr
1               5                   10                  15

Gln Thr Lys Lys Glu Thr Phe Asn Cys Ser Glu Tyr Gly Asn Arg Ser
            20                  25                  30

Cys Pro Glu Asn Glu Arg Ser Leu Gly Val Arg Val Ala Met Tyr Ser
        35                  40                  45

Phe Met Ala Gly Ser Ile Phe Ile Thr Ile Phe Gly Asn Leu Ala Val
    50                  55                  60

Ile Ile Ser Ile Ser Tyr Phe Lys Gln Leu His Thr Pro Thr Asn Phe
65                  70                  75                  80

Leu Ile Leu Ser Met Ala Ile Thr Asp Phe Leu Leu Gly Phe Thr Ile
                85                  90                  95

Met Pro Tyr Ser Met Ile Arg Ser Val Glu Asn Cys Trp Tyr Phe Gly
            100                 105                 110

Leu Thr Phe Cys Lys Ile His Tyr Ser Phe Asp Leu Met Leu Ser Ile
        115                 120                 125

Thr Ser Ile Phe His Leu Cys Ser Val Ala Ile Asp Arg Phe Tyr Ala
    130                 135                 140

Val Cys Tyr Pro Leu Arg Tyr Ser Thr Lys Ile Thr Ile Pro Val Ile
145                 150                 155                 160

Lys Arg Leu Leu Leu Leu Cys Trp Ser Val Pro Gly Ala Phe Ala Phe
                165                 170                 175

Gly Val Val Phe Ser Glu Ala Tyr Ala Asp Gly Ile Glu Gly Tyr Asp
            180                 185                 190

Ile Leu Val Ala Cys Ser Ser Ser Cys Pro Val Met Phe Asn Lys Leu
        195                 200                 205

Trp Gly Thr Thr Leu Phe Met Ala Gly Phe Phe Thr Pro Gly Ser Met
    210                 215                 220

Met Val Gly Ile Tyr Gly Lys Ile Phe Ala Val Ser Arg Lys His Ala
225                 230                 235                 240

His Ala Ile Asn Asn Leu Gln Glu Asn Gln Asn Asn Gln Val Lys Lys
                245                 250                 255

Asp Lys Lys Ala Ala Lys Thr Leu Gly Ile Val Ile Gly Val Phe Leu
            260                 265                 270

Leu Cys Trp Phe Pro Ser Phe Phe Thr Ile Leu Leu Asp Pro Phe Leu
        275                 280                 285

Asn Phe Ser Thr Pro Val Val Leu Phe Asp Ala Leu Thr Trp Phe Gly
    290                 295                 300

Tyr Phe Asn Ser Thr Cys Asn Pro Leu Ile Tyr Gly Phe Phe Tyr Pro
305                 310                 315                 320

Trp Phe Arg Arg Ala Leu Lys Tyr Ile Leu Leu Gly Lys Ile Phe Ser
                325                 330                 335

Ser Cys Phe His Thr Thr Asn Leu Cys Met Gln Lys Glu Ser Glu
            340                 345                 350

<210>  7
<211>  1030
<212>  DNA
<213>  Pan troglodytes

<220>
<221>  ptTAAR3 Y
<222>  (1)..(1030)
<223>  pseudogene

<400>  7
atggatctaa cttatattcc cgacgaccta tccagttgtc caaaatttgt aaataaatcc      60

tgtcctccca ccaaccgctc ttttcatgtc cgggtgataa tgtattcggt tatgactgga     120

gccatgatta tccctattcg gaaacttggt tataatggtt tccatatcgc atttcaaaca     180

gcttcactct cccacaaact ttctgatcct ctccatggca accacggact ttctgctggg     240

ttttgtcatt atgccataca acataatgcg atcagtggag agttgctggt actttgggga     300

tggcttttgt aaattccaca caagcttaga catgatgctc agcctgacct ccattttcca     360

cctctgttcc attgctattg accgatttta tgccgtgtgt taccctttac attacacaac     420

cagaacgaca aactccacca taaagcaact gctggcattt tgctggtcag ttcctgctct 480

tttttctttt ggtttagttc tatccgaggc caatgtttcc ggtatgcaga gctataagat 540

acttgttgct tgcttcaatt actgtgccct tactttcaac aaattctggg ggacaatatt 600

gttcactaca tgtttcttta cccctggctc catcatggtt ggtatttatg gcaaaatctt 660

tatcgtttcc aaacagcatg ctcgagtcat cagccatgtg cctgaaaaca caaagggggc 720

agtgaaaaaa cacctatcca agaaaaagga caggaaagca gcgaagacac tgggtatagt 780

aatgggggtg tttctggctt gctggttgcc ttgttttctt gctgttctga ttgacccata 840

cctagactac tccactccca tactaatatt ggatctttta gtgtggctcg ggtacttcaa 900

ctctacttgc aaccctctta ttcatggctt tttttatcca tggtttcaga aagcactcaa 960

gtacatagtg tcaggaaaaa tatttagctc ccattcagaa actgcaaatt tgtttcctga 1020

agcacattaa 1030

<210> 8
<211> 1032
<212> DNA
<213> Pan troglodytes

<220>
<221> ptTAAR3 Yf
<222> (1)..(1032)
<223> fixed pseudogene

<400> 8
atggatctaa cttatattcc cgacgaccta tccagttgtc caaaatttgt aaataaatcc 60

tgtcctccca ccaaccgctc ttttcatgtc cgggtgataa tgtattcggt tatgactgga 120

gccatgatta tccccatatt cggaaacttg gttataatgg tttccatatc gcatttcaaa 180

cagcttcact ctcccacaaa ctttctgatc ctctccatgg caaccacgga ctttctgctg 240

ggttttgtca ttatgccata caacataatg cgatcagtgg agagttgctg gtactttggg 300

gatggctttt gtaaattcca cacaagctta gacatgatgc tcagcctgac ctccattttc 360

cacctctgtt ccattgctat tgaccgattt tatgccgtgt gttacccttt acattacaca 420

accagaacga caaactccac cataaagcaa ctgctggcat tttgctggtc agttcctgct 480

cttttttctt ttggtttagt tctatccgag gccaatgttt ccggtatgca gagctataag 540

atacttgttg cttgcttcaa ttactgtgcc cttactttca acaaattctg ggggacaata 600

ttgttcacta catgtttctt tacccctggc tccatcatgg ttggtattta tggcaaaatc 660

tttatcgttt ccaaacagca tgctcgagtc atcagccatg tgcctgaaaa cacaaagggg 720

gcagtgaaaa aacacctatc caagaaaaag gacaggaaag cagcgaagac actgggtata 780

gtaatggggg tgtttctggc ttgctggttg ccttgttttc ttgctgttct gattgaccca 840

tacctagact actccactcc catactaata ttggatcttt tagtgtggct cgggtacttc 900

aactctactt gcaaccctct tattcatggc tttttttatc catggtttca gaaagcactc 960

aagtacatag tgtcaggaaa aatatttagc tcccattcag aaactgcaaa tttgtttcct 1020

gaagcacatt aa                                                          1032

<210>  9
<211>  343
<212>  PRT
<213>  Pan troglodytes

<220>
<221>  ptTAAR3 Yf
<222>  (1)..(343)
<223>  protein of fixed pseudogene

<400>  9

Met Asp Leu Thr Tyr Ile Pro Asp Asp Leu Ser Ser Cys Pro Lys Phe
1               5                   10                  15

Val Asn Lys Ser Cys Pro Pro Thr Asn Arg Ser Phe His Val Arg Val
            20                  25                  30

Ile Met Tyr Ser Val Met Thr Gly Ala Met Ile Ile Pro Ile Phe Gly
        35                  40                  45

Asn Leu Val Ile Met Val Ser Ile Ser His Phe Lys Gln Leu His Ser
    50                  55                  60

Pro Thr Asn Phe Leu Ile Leu Ser Met Ala Thr Thr Asp Phe Leu Leu
65                  70                  75                  80

Gly Phe Val Ile Met Pro Tyr Asn Ile Met Arg Ser Val Glu Ser Cys
                85                  90                  95

Trp Tyr Phe Gly Asp Gly Phe Cys Lys Phe His Thr Ser Leu Asp Met
            100                 105                 110

Met Leu Ser Leu Thr Ser Ile Phe His Leu Cys Ser Ile Ala Ile Asp
        115                 120                 125

Arg Phe Tyr Ala Val Cys Tyr Pro Leu His Tyr Thr Thr Arg Thr Thr
    130                 135                 140

Asn Ser Thr Ile Lys Gln Leu Leu Ala Phe Cys Trp Ser Val Pro Ala
145                 150                 155                 160

Leu Phe Ser Phe Gly Leu Val Leu Ser Glu Ala Asn Val Ser Gly Met
                165                 170                 175

Gln Ser Tyr Lys Ile Leu Val Ala Cys Phe Asn Tyr Cys Ala Leu Thr
            180                 185                 190

Phe Asn Lys Phe Trp Gly Thr Ile Leu Phe Thr Thr Cys Phe Phe Thr
        195                 200                 205

Pro Gly Ser Ile Met Val Gly Ile Tyr Gly Lys Ile Phe Ile Val Ser
    210                 215                 220

Lys Gln His Ala Arg Val Ile Ser His Val Pro Glu Asn Thr Lys Gly
225                 230                 235                 240

Ala Val Lys Lys His Leu Ser Lys Lys Lys Asp Arg Lys Ala Ala Lys
                245                 250                 255

Thr Leu Gly Ile Val Met Gly Val Phe Leu Ala Cys Trp Leu Pro Cys
            260                 265                 270

Phe Leu Ala Val Leu Ile Asp Pro Tyr Leu Asp Tyr Ser Thr Pro Ile
        275                 280                 285

Leu Ile Leu Asp Leu Leu Val Trp Leu Gly Tyr Phe Asn Ser Thr Cys
    290                 295                 300

Asn Pro Leu Ile His Gly Phe Phe Tyr Pro Trp Phe Gln Lys Ala Leu
305                 310                 315                 320

Lys Tyr Ile Val Ser Gly Lys Ile Phe Ser Ser His Ser Glu Thr Ala
                325                 330                 335

Asn Leu Phe Pro Glu Ala His
            340

<210>  10
<211>  1049
<212>  DNA
<213>  Pan troglodytes

<220>
<221>  ptTAAR4 Y
<222>  (1)..(1049)
<223>  pseudogene

<400>  10
atgatgaatt tgcctgaccc tcagaacccc ccaacagtac aattttgctt tagttcagtt      60

aacaattcat gccctagaaa tgtgaggcca gtgcagagtg tctgggccat gtacctggtc     120

atgatcgggt ctatagtgat gacaatgctg ggcaacatga tcgtaatgat ttccatcgct     180

cacttcaagc agctccactc cccgaccaac ttcttgatcc tctccatggc catcactgac     240

tttttgctga gctgtgtggt catgcccttc agtgtgatca gatccattga gtcctgctgg     300

tattttggag acctcttttg caaagtccac agctgctgtg acatcatgct ctgcaccacc     360

tccatttttc acctctgcct catctcagtt gaccgttact atgctgtttg cgacccattg     420

caatatgtca ccagaattac catccctgtc atagaactct ttctactcat cagttggtcc     480

attcccatct tttttgcctt tggcctggta ttctcaaaac taaacataat tggtgcagaa     540

gagtttgttg cagccattga ttgcacaggt ttgtgtgtgt taatatttaa taagctctgg     600

gggggtactg gcctccttta tagctttctt tctccccggg acaaccatgg tgggaattta     660

catacatatt tttacagtag ccaggaagca tgccatgcaa attggcacag gttctaggac     720

taaacaggct gggtcaaaaa gcaaaaaaaa agtcatcctc taaaacagaa agcaaggcca 780

ccaggacctt aggcatagtc atgggagtgt ttgtgttgtg ctggctgccc ttctttgtct 840

tgacgatcac aaatcctttc attaatttta caacccctga agatctgtac aatgtcttcc 900

tctggctggg ctatttcaac tccgctttca accccatttt atatggcatg ttttatcctt 960

ggtttcgcaa ggcattgagg atgattgtca caggcatgat cttcctccct gactcttcca 1020

ccctaagcct gttttctgcc catgcttag 1049

<210> 11
<211> 1047
<212> DNA
<213> Pan troglodytes

<220>
<221> ptTAAR4 Yf
<222> (1)..(1047)
<223> fixed pseudogene

<400> 11
atgatgaatt tgcctgaccc tcagaacccc ccaacagtac aattttgctt tagttcagtt 60

aacaattcat gccctagaaa tgtgaggcca gtgcagagtg tctgggccat gtacctggtc 120

atgatcgggt ctatagtgat gacaatgctg ggcaacatga tcgtaatgat ttccatcgct 180

cacttcaagc agctccactc cccgaccaac ttcttgatcc tctccatggc catcactgac 240

tttttgctga gctgtgtggt catgcccttc agtgtgatca gatccattga gtcctgctgg 300

tattttggag acctcttttg caaagtccac agctgctgtg acatcatgct ctgcaccacc 360

tccatttttc acctctgcct catctcagtt gaccgttact atgctgtttg cgacccattg 420

caatatgtca ccagaattac catccctgtc atagaactct ttctactcat cagttggtcc 480

attcccatct tttttgcctt tggcctggta ttctcaaaac taaacataat tggtgcagaa 540

gagtttgttg cagccattga ttgcacaggt ttgtgtgtgt taatatttaa taagctctgg 600

ggggtactgg cctcctttat agctttcttt ctccccggga caaccatggt gggaatttac 660

atacatattt ttacagtagc caggaagcat gccatgcaaa ttggcacagg ttctaggact 720

aaacaggctg ggtcaaaaag caaaaaaaag tcatcctcta aaacagaaag caaggccacc 780

aggaccttag gcatagtcat gggagtgttt gtgttgtgct ggctgccctt ctttgtcttg 840

acgatcacaa atcctttcat taattttaca acccctgaag atctgtacaa tgtcttcctc 900

tggctgggct atttcaactc cgctttcaac cccattttat atggcatgtt ttatccttgg 960

tttcgcaagg cattgaggat gattgtcaca ggcatgatct tcctccctga ctcttccacc 1020

ctaagcctgt tttctgccca tgcttag 1047

<210> 12
<211> 348
<212> PRT
<213> Pan troglodytes

<220>
<221> ptTAAR4 Yf

<222>  (1)..(348)
<223>  protein of fixed pseudogen

<400>  12

Met Met Asn Leu Pro Asp Pro Gln Asn Pro Pro Thr Val Gln Phe Cys
1               5                   10                  15

Phe Ser Ser Val Asn Asn Ser Cys Pro Arg Asn Val Arg Pro Val Gln
            20                  25                  30

Ser Val Trp Ala Met Tyr Leu Val Met Ile Gly Ser Ile Val Met Thr
        35                  40                  45

Met Leu Gly Asn Met Ile Val Met Ile Ser Ile Ala His Phe Lys Gln
    50                  55                  60

Leu His Ser Pro Thr Asn Phe Leu Ile Leu Ser Met Ala Ile Thr Asp
65                  70                  75                  80

Phe Leu Leu Ser Cys Val Val Met Pro Phe Ser Val Ile Arg Ser Ile
                85                  90                  95

Glu Ser Cys Trp Tyr Phe Gly Asp Leu Phe Cys Lys Val His Ser Cys
            100                 105                 110

Cys Asp Ile Met Leu Cys Thr Thr Ser Ile Phe His Leu Cys Leu Ile
        115                 120                 125

Ser Val Asp Arg Tyr Tyr Ala Val Cys Asp Pro Leu Gln Tyr Val Thr
    130                 135                 140

Arg Ile Thr Ile Pro Val Ile Glu Leu Phe Leu Leu Ile Ser Trp Ser
145                 150                 155                 160

Ile Pro Ile Phe Phe Ala Phe Gly Leu Val Phe Ser Lys Leu Asn Ile
                165                 170                 175

Ile Gly Ala Glu Glu Phe Val Ala Ala Ile Asp Cys Thr Gly Leu Cys
            180                 185                 190

Val Leu Ile Phe Asn Lys Leu Trp Gly Val Leu Ala Ser Phe Ile Ala
        195                 200                 205

Phe Phe Leu Pro Gly Thr Thr Met Val Gly Ile Tyr Ile His Ile Phe
    210                 215                 220

Thr Val Ala Arg Lys His Ala Met Gln Ile Gly Thr Gly Ser Arg Thr
225                 230                 235                 240

Lys Gln Ala Gly Ser Lys Ser Lys Lys Lys Ser Ser Ser Lys Thr Glu
                245                 250                 255

Ser Lys Ala Thr Arg Thr Leu Gly Ile Val Met Gly Val Phe Val Leu

260                 265                 270

Cys Trp Leu Pro Phe Phe Val Leu Thr Ile Thr Asn Pro Phe Ile Asn
        275                 280                 285

Phe Thr Thr Pro Glu Asp Leu Tyr Asn Val Phe Leu Trp Leu Gly Tyr
    290                 295                 300

Phe Asn Ser Ala Phe Asn Pro Ile Leu Tyr Gly Met Phe Tyr Pro Trp
305                 310                 315                 320

Phe Arg Lys Ala Leu Arg Met Ile Val Thr Gly Met Ile Phe Leu Pro
                325                 330                 335

Asp Ser Ser Thr Leu Ser Leu Phe Ser Ala His Ala
            340                 345

<210>  13
<211>  1014
<212>  DNA
<213>  Pan troglodytes

<220>
<221>  ptTAAR5
<222>  (1)..(1014)

<400>  13
atgagagctg tcttcatcca aggtgctgaa gagcaccctg cggcattctg ctaccaggtg      60

aatgggtctt gccccaggac agtacatact ctgggcatcc agttggtcat ctacctggcc     120

tgtgcagcag gcatgctgat tatcgtgcta gggaatttat ttgtggcatt tgctgtgtcc     180

tacttcaaag cgcttcacac gcccaccaac ttcttgctgc tctccctggc cctggctgac     240

atgtttctgg gtctgctggt gctgcccctc agcaccattc gctcagtgga gagctgctgg     300

ttcttcgggg acttcctctg ccgcctgcac acctacctgg accccctctt ctgcctcacc     360

tccatcttcc atctctgttt catttccatt gaccgccact gtgccatctg tgaccccctg     420

ctctatccct ccaagttcac agtgagggtg gctctcaggt acatcctggc aggatggggg     480

gtgcccgcag catacacttc cttattcctc tacacagatg tggtagagac aaggctcagc     540

cagtggctgg aagagatgcc ttgtgtgggc agttgccagc tgctgctcaa taaattttgg     600

ggctggttaa acttcccttt gttctttgtc ccctgcctca ttatgatcag cttgtatgtg     660

aagatctttg tggttgctac cagacaggct cagcagatta ccacattgag caaaaacctg     720

gctggggctg ccaagcatga cagaaaagct gccaagaccc tgggcattgc tgtgggcata     780

tacctcttgt gctggctgcc cttcaccata gacacgatgg tcgacagcct ccttcacttt     840

atcacacccc cacttgtctt tgacatcttt atctggtttg cttacttcaa ctcagcctgc     900

aatcccatca tctatgtctt ttcctaccag tggtttcgga aggcactgaa actgacgctg     960

agccagaagg tcttctcacc gcagacacgc actgttgatt tgtaccaaga atga          1014

<210>  14

<211>  337
<212>  PRT
<213>  Pan troglodytes


<220>
<221>  ptTAAR5
<222>  (1)..(337)

<400>  14

Met Arg Ala Val Phe Ile Gln Gly Ala Glu Glu His Pro Ala Ala Phe
1               5                   10                  15


Cys Tyr Gln Val Asn Gly Ser Cys Pro Arg Thr Val His Thr Leu Gly
            20                  25                  30


Ile Gln Leu Val Ile Tyr Leu Ala Cys Ala Ala Gly Met Leu Ile Ile
        35                  40                  45


Val Leu Gly Asn Leu Phe Val Ala Phe Ala Val Ser Tyr Phe Lys Ala
    50                  55                  60


Leu His Thr Pro Thr Asn Phe Leu Leu Leu Ser Leu Ala Leu Ala Asp
65                  70                  75                  80


Met Phe Leu Gly Leu Leu Val Leu Pro Leu Ser Thr Ile Arg Ser Val
                85                  90                  95


Glu Ser Cys Trp Phe Phe Gly Asp Phe Leu Cys Arg Leu His Thr Tyr
            100                 105                 110


Leu Asp Pro Leu Phe Cys Leu Thr Ser Ile Phe His Leu Cys Phe Ile
        115                 120                 125


Ser Ile Asp Arg His Cys Ala Ile Cys Asp Pro Leu Leu Tyr Pro Ser
    130                 135                 140


Lys Phe Thr Val Arg Val Ala Leu Arg Tyr Ile Leu Ala Gly Trp Gly
145                 150                 155                 160


Val Pro Ala Ala Tyr Thr Ser Leu Phe Leu Tyr Thr Asp Val Val Glu
                165                 170                 175


Thr Arg Leu Ser Gln Trp Leu Glu Glu Met Pro Cys Val Gly Ser Cys
            180                 185                 190


Gln Leu Leu Leu Asn Lys Phe Trp Gly Trp Leu Asn Phe Pro Leu Phe
        195                 200                 205


Phe Val Pro Cys Leu Ile Met Ile Ser Leu Tyr Val Lys Ile Phe Val
    210                 215                 220


Val Ala Thr Arg Gln Ala Gln Gln Ile Thr Thr Leu Ser Lys Asn Leu
225                 230                 235                 240

Ala Gly Ala Ala Lys His Asp Arg Lys Ala Ala Lys Thr Leu Gly Ile
                245                 250                 255

Ala Val Gly Ile Tyr Leu Leu Cys Trp Leu Pro Phe Thr Ile Asp Thr
            260                 265                 270

Met Val Asp Ser Leu Leu His Phe Ile Thr Pro Pro Leu Val Phe Asp
        275                 280                 285

Ile Phe Ile Trp Phe Ala Tyr Phe Asn Ser Ala Cys Asn Pro Ile Ile
    290                 295                 300

Tyr Val Phe Ser Tyr Gln Trp Phe Arg Lys Ala Leu Lys Leu Thr Leu
305                 310                 315                 320

Ser Gln Lys Val Phe Ser Pro Gln Thr Arg Thr Val Asp Leu Tyr Gln
                325                 330                 335

Glu

<210>  15
<211>  1038
<212>  DNA
<213>  Pan troglodytes

<220>
<221>  ptTAAR6
<222>  (1)..(1038)

<400>  15
atgagcagca actcatccct gctggtggct gtgcagctgt gctacccgaa cgtgaatggg      60

tcctgtgtgg aaaccctcta ctcgcctgga tcccgggtga ttctgtacat agtgtttggc     120

tttggggctg tgttggctgt gtttggaaac ctcctggtga tgatttcaat cctccatttc     180

aagcagctgc actctccgac caattttctc gttgcctctc tggcctgcgc tgatttctta     240

gtgggtgtga ctgtgatgcc cttcagcatg gtcaggaccg tggagagctg ctggtatttt     300

gggagaagtt tttgtacttt ccacacctgc tgtgatgtgg cattttgtta ctcttctctc     360

tttcacttgt gcttcatctc catcgacagg tacattgcgg ttactgaccc cctggtctat     420

cctaccaagt tcaccgtatc tgtgtcagga atttgcatca gcgtgtcctg gatcctgccc     480

ctcatgtaca gcggtgctgt gttctacaca ggtgtctatg acgatgggct ggaggaatta     540

tctgatgccc taaactgtat aggaggttgt cagaccgttg taaatcaaaa ctgggtgttg     600

atagattgtc tatccttctt tatacctacc tttattatga taattctgta tggtaacata     660

tttcttgtgg ctagacgaca ggcgaaaaag atagaaaata ctggtagcaa gacagaatca     720

tcctcagaga gttacaaagc cagagtggcc aggagagaga gaaaagcagc taaaaccctg     780

ggggtcacag tggtagcatt tatgatttca tggttaccat atagcattga ttcattaatt     840

gatgccttta tgggctttat aacccctgcc tatatttatg agatttgctg ttggtgtgct     900

tattataact cagccatgaa tcctttgatt tatgctttat tttacccatg gtttaggaaa    960

gcaataaaag ttattgtaac tggtcaggtt ttaaagaaca gttcagcaac catgaatttg   1020

ttttctgaac atatataa                                                 1038

<210>  16
<211>  345
<212>  PRT
<213>  Pan troglodytes

<220>
<221>  ptTAAR6
<222>  (1)..(345)

<400>  16

Met Ser Ser Asn Ser Ser Leu Leu Val Ala Val Gln Leu Cys Tyr Pro
1               5                   10                  15

Asn Val Asn Gly Ser Cys Val Glu Thr Leu Tyr Ser Pro Gly Ser Arg
            20                  25                  30

Val Ile Leu Tyr Ile Val Phe Gly Phe Gly Ala Val Leu Ala Val Phe
        35                  40                  45

Gly Asn Leu Leu Val Met Ile Ser Ile Leu His Phe Lys Gln Leu His
    50                  55                  60

Ser Pro Thr Asn Phe Leu Val Ala Ser Leu Ala Cys Ala Asp Phe Leu
65                  70                  75                  80

Val Gly Val Thr Val Met Pro Phe Ser Met Val Arg Thr Val Glu Ser
                85                  90                  95

Cys Trp Tyr Phe Gly Arg Ser Phe Cys Thr Phe His Thr Cys Cys Asp
            100                 105                 110

Val Ala Phe Cys Tyr Ser Ser Leu Phe His Leu Cys Phe Ile Ser Ile
        115                 120                 125

Asp Arg Tyr Ile Ala Val Thr Asp Pro Leu Val Tyr Pro Thr Lys Phe
    130                 135                 140

Thr Val Ser Val Ser Gly Ile Cys Ile Ser Val Ser Trp Ile Leu Pro
145                 150                 155                 160

Leu Met Tyr Ser Gly Ala Val Phe Tyr Thr Gly Val Tyr Asp Asp Gly
                165                 170                 175

Leu Glu Glu Leu Ser Asp Ala Leu Asn Cys Ile Gly Gly Cys Gln Thr
            180                 185                 190

Val Val Asn Gln Asn Trp Val Leu Ile Asp Cys Leu Ser Phe Phe Ile
        195                 200                 205

Pro Thr Phe Ile Met Ile Ile Leu Tyr Gly Asn Ile Phe Leu Val Ala
    210                 215                 220

Arg Arg Gln Ala Lys Lys Ile Glu Asn Thr Gly Ser Lys Thr Glu Ser
225                 230                 235                 240

Ser Ser Glu Ser Tyr Lys Ala Arg Val Ala Arg Arg Glu Arg Lys Ala
                245                 250                 255

Ala Lys Thr Leu Gly Val Thr Val Val Ala Phe Met Ile Ser Trp Leu
            260                 265                 270

Pro Tyr Ser Ile Asp Ser Leu Ile Asp Ala Phe Met Gly Phe Ile Thr
        275                 280                 285

Pro Ala Tyr Ile Tyr Glu Ile Cys Cys Trp Cys Ala Tyr Tyr Asn Ser
    290                 295                 300

Ala Met Asn Pro Leu Ile Tyr Ala Leu Phe Tyr Pro Trp Phe Arg Lys
305                 310                 315                 320

Ala Ile Lys Val Ile Val Thr Gly Gln Val Leu Lys Asn Ser Ser Ala
                325                 330                 335

Thr Met Asn Leu Phe Ser Glu His Ile
            340                 345

<210> 17
<211> 1027
<212> DNA
<213> Pan troglodytes

<220>
<221> ptTAAR8 Y
<222> (1)..(1027)
<223> pseudogene

<400> 17
atgaccagca atttttccta acctgttgtg cagctttgct atgaggatgt gaatggatct 60

tgtattaaaa ctccctattc tcctgggccc tgggtgattc tgtacacggc gtttagcttt 120

gggtctttgc tggctgtatt tgcaaatctc ttagtaatga cttctgttct tcattttaag 180

cagctgcact ctccaaccaa ttttctcatt gcctctctgg cctgtgctga cttcttggta 240

ggtgtgactg tgatgccctt cagcatggtc aggacggtgg agagctgctg gtattttgga 300

gccaaatttt gtactcttca cagttgctgt gatgtgtcat tttgttactc ttctgtcctc 360

cacttgtgct tcatctgcat cgacaggtac attgcggtta ctgagcccct ggtctatgct 420

accaagttca ccgtgtccgt gtcgggaatt tgcatcagcg tgtgctggat tctgcctctc 480

acgtacagcg gtgctgtgtt ctacacaggt gtcaatgatg atgagctggg ggagttagta 540

agtgctctca actgtgtagg tggctgtcaa attgttgtaa gtcaaggctg ggtgttgata 600

gattttctgt tattcttcat acctaccctt gttatgataa ttctttaccg taagattttt 660

cttatagcta aacaacaagc tataaaaatt gaaactacta gtagcaaagt agaatcatcc 720

tcagagagtt ataaaatcag agtggccgag agagaggaaa gcagctaaaa ccctgggggt 780

cacggtacta gcatttgtta tttcatggtt accgtataca gttgatatat taatcgatgc 840

ctttatgggc ttcctgaccc ctgcctatat ctatgaaatt tgctgttgga gtgcttattg 900

taactcagcc atgaatcctt tgatttatgc tttattttat ccttggttta ggaaagccat 960

aaaacttatt ttaagtgggg atgttttaaa ggctagttca tcagccatta gtttattttc 1020

agaataa 1027

<210> 18
<211> 1029
<212> DNA
<213> Pan troglodytes

<220>
<221> ptTAAR8 Yf
<222> (1)..(1029)
<223> fixed pseudogene

<400> 18
atgaccagca atttttccta tcctgttgtg cagctttgct atgaggatgt gaatggatct 60

tgtattaaaa ctccctattc tcctgggccc tgggtgattc tgtacacggc gtttagcttt 120

gggtctttgc tggctgtatt tgcaaatctc ttagtaatga cttctgttct tcattttaag 180

cagctgcact ctccaaccaa ttttctcatt gcctctctgg cctgtgctga cttcttggta 240

ggtgtgactg tgatgccctt cagcatggtc aggacggtgg agagctgctg gtattttgga 300

gccaaatttt gtactcttca cagttgctgt gatgtgtcat tttgttactc ttctgtcctc 360

cacttgtgct tcatctgcat cgacaggtac attgcggtta ctgagcccct ggtctatgct 420

accaagttca ccgtgtccgt gtcgggaatt tgcatcagcg tgtgctggat tctgcctctc 480

acgtacagcg gtgctgtgtt ctacacaggt gtcaatgatg atgagctggg ggagttagta 540

agtgctctca actgtgtagg tggctgtcaa attgttgtaa gtcaaggctg ggtgttgata 600

gattttctgt tattcttcat acctaccctt gttatgataa ttctttaccg taagattttt 660

cttatagcta aacaacaagc tataaaaatt gaaactacta gtagcaaagt agaatcatcc 720

tcagagagtt ataaaatcag agtggccaag agagagagga aagcagctaa aaccctgggg 780

gtcacggtac tagcatttgt tatttcatgg ttaccgtata cagttgatat attaatcgat 840

gcctttatgg gcttcctgac ccctgcctat atctatgaaa tttgctgttg gagtgcttat 900

tgtaactcag ccatgaatcc tttgatttat gctttatttt atccttggtt taggaaagcc 960

ataaaactta ttttaagtgg ggatgtttta aaggctagtt catcagccat tagtttattt 1020

tcagaataa 1029

<210> 19
<211> 342
<212> PRT
<213> Pan troglodytes

<220>
<221>  ptTAAR8 Yf
<222>  (1)..(342)
<223>  protein of fixed pseudogene

<400>  19

Met Thr Ser Asn Phe Ser Tyr Pro Val Val Gln Leu Cys Tyr Glu Asp
1               5                   10                  15

Val Asn Gly Ser Cys Ile Lys Thr Pro Tyr Ser Pro Gly Pro Trp Val
            20                  25                  30

Ile Leu Tyr Thr Ala Phe Ser Phe Gly Ser Leu Leu Ala Val Phe Ala
        35                  40                  45

Asn Leu Leu Val Met Thr Ser Val Leu His Phe Lys Gln Leu His Ser
    50                  55                  60

Pro Thr Asn Phe Leu Ile Ala Ser Leu Ala Cys Ala Asp Phe Leu Val
65                  70                  75                  80

Gly Val Thr Val Met Pro Phe Ser Met Val Arg Thr Val Glu Ser Cys
                85                  90                  95

Trp Tyr Phe Gly Ala Lys Phe Cys Thr Leu His Ser Cys Cys Asp Val
            100                 105                 110

Ser Phe Cys Tyr Ser Ser Val Leu His Leu Cys Phe Ile Cys Ile Asp
        115                 120                 125

Arg Tyr Ile Ala Val Thr Glu Pro Leu Val Tyr Ala Thr Lys Phe Thr
    130                 135                 140

Val Ser Val Ser Gly Ile Cys Ile Ser Val Cys Trp Ile Leu Pro Leu
145                 150                 155                 160

Thr Tyr Ser Gly Ala Val Phe Tyr Thr Gly Val Asn Asp Asp Glu Leu
                165                 170                 175

Gly Glu Leu Val Ser Ala Leu Asn Cys Val Gly Gly Cys Gln Ile Val
            180                 185                 190

Val Ser Gln Gly Trp Val Leu Ile Asp Phe Leu Leu Phe Phe Ile Pro
        195                 200                 205

Thr Leu Val Met Ile Ile Leu Tyr Arg Lys Ile Phe Leu Ile Ala Lys
    210                 215                 220

Gln Gln Ala Ile Lys Ile Glu Thr Thr Ser Ser Lys Val Glu Ser Ser
225                 230                 235                 240

Ser Glu Ser Tyr Lys Ile Arg Val Ala Lys Arg Glu Arg Lys Ala Ala
                245                 250                 255

Lys Thr Leu Gly Val Thr Val Leu Ala Phe Val Ile Ser Trp Leu Pro
            260                 265                 270

Tyr Thr Val Asp Ile Leu Ile Asp Ala Phe Met Gly Phe Leu Thr Pro
        275                 280                 285

Ala Tyr Ile Tyr Glu Ile Cys Cys Trp Ser Ala Tyr Cys Asn Ser Ala
    290                 295                 300

Met Asn Pro Leu Ile Tyr Ala Leu Phe Tyr Pro Trp Phe Arg Lys Ala
305                 310                 315                 320

Ile Lys Leu Ile Leu Ser Gly Asp Val Leu Lys Ala Ser Ser Ser Ala
                325                 330                 335

Ile Ser Leu Phe Ser Glu
            340

<210>  20
<211>  1048
<212>  DNA
<213>  Pan troglodytes

<220>
<221>  ptTAAR9 Y
<222>  (1)..(1048)
<223>  pseudogene

<400>  20
   atggtgaaca atttctccca agctgaggct gtggagctgt gttacaagaa cgtgaacaga      60
   tcctgcatta aaactcctta ctcgccaggt cctcgagcta tcctctatgc tgtccttggt     120
   tttggggctg tgctgtcagt gtttggaaac ttactggtca tgattgctat ccttcacttc     180
   aaacaactgc acacacctac aaactttctg atcatgtcgc tggcctgtgc tgacttcttg     240
   gtgggagtca ctgtgatgcc cttcagcaca gtgaggtctg tgtagaactg ttggtacttt     300
   ggggacagtt actgtaaatt ccatacatgt tttgacacat ccttctgttt tgcttcttta     360
   tttcatttat gccgtatctc tgttgataga tacattgctg ttactgatcc tctgacctat     420
   ccaaccaagt ttactgtgtc aatttcagga atatgcattg ttctttcctg gttcttttct     480
   gtcacataca gcttttcgat cttttacacg ggagccaacg aagaaggaat tgaggaatta     540
   gtagttgctc taacctgtgt aggaggctgc caggctccac tgaatcaaaa ctgggtccta     600
   ctttgttttc ttctattctt tatacccact gtcgctatgg tgtttatata cagtaagata     660
   tttttggtgg ccaagcatca ggctaggaag atagaaagta cagccagcca agctcagtcc     720
   tcctcagaga gttacaagga aagagtagca aaaagaaaga gaaaggctgc caaaaccttg     780
   ggaattgcta tggcagcatt tcttgtctct tggctaccat acatcgttga tgcagtgatt     840
   gatgcttaca tgaattttat aactcctcct tatgtttatg agattttagt ttggtgtgtt     900
   tattataatt cagctatgaa ccccttgatt tatgctttct tttacccatg gtttcggaag     960
   gcaataaaac ttatttgtaa gcggcaaggt cttaaggagt gattcatcaa caactaattt 1020
   attttctgaa gaagtagaga aagatcaa 1048

<210> 21
<211> 1047
<212> DNA
<213> Pan troglodytes

<220>
<221> ptTAAR9 Yf
<222> (1)..(1047)
<223> fixed pseudogene

<400> 21
atggtgaaca atttctccca agctgaggct gtggagctgt gttacaagaa cgtgaacaga 60

tcctgcatta aaactcctta ctcgccaggt cctcgagcta tcctctatgc tgtccttggt 120

tttggggctg tgctgtcagt gtttggaaac ttactggtca tgattgctat ccttcacttc 180

aaacaactgc acacacctac aaactttctg atcatgtcgc tggcctgtgc tgacttcttg 240

gtgggagtca ctgtgatgcc cttcagcaca gtgaggtctg tgtacaactg ttggtacttt 300

ggggacagtt actgtaaatt ccatacatgt tttgacacat ccttctgttt tgcttcttta 360

tttcatttat gccgtatctc tgttgataga tacattgctg ttactgatcc tctgacctat 420

ccaaccaagt ttactgtgtc aatttcagga atatgcattg ttctttcctg gttcttttct 480

gtcacataca gcttttcgat cttttacacg ggagccaacg aagaaggaat tgaggaatta 540

gtagttgctc taacctgtgt aggaggctgc caggctccac tgaatcaaaa ctgggtccta 600

ctttgttttc ttctattctt tatacccact gtcgctatgg tgtttatata cagtaagata 660

tttttggtgg ccaagcatca ggctaggaag atagaaagta cagccagcca agctcagtcc 720

tcctcagaga gttacaagga aagagtagca aaaagaaaga gaaaggctgc caaaaccttg 780

ggaattgcta tggcagcatt tcttgtctct tggctaccat acatcgttga tgcagtgatt 840

gatgcttaca tgaattttat aactcctcct tatgtttatg agattttagt ttggtgtgtt 900

tattataatt cagctatgaa ccccttgatt tatgctttct tttacccatg gtttcggaag 960

gcaataaaac ttattgtaag cggcaaggtc ttaaggagtg attcatcaac aactaattta 1020

ttttctgaag aagtagagaa agatcaa 1047

<210> 22
<211> 349
<212> PRT
<213> Pan troglodytes

<220>
<221> ptTAAR9 Yf
<222> (1)..(349)
<223> protein of fixed pseudogene

<400> 22

Met Val Asn Asn Phe Ser Gln Ala Glu Ala Val Glu Leu Cys Tyr Lys
1               5                   10                  15

Asn Val Asn Arg Ser Cys Ile Lys Thr Pro Tyr Ser Pro Gly Pro Arg
            20                  25                  30

Ala Ile Leu Tyr Ala Val Leu Gly Phe Gly Ala Val Leu Ser Val Phe
        35                  40                  45

Gly Asn Leu Leu Val Met Ile Ala Ile Leu His Phe Lys Gln Leu His
    50                  55                  60

Thr Pro Thr Asn Phe Leu Ile Met Ser Leu Ala Cys Ala Asp Phe Leu
65                  70                  75                  80

Val Gly Val Thr Val Met Pro Phe Ser Thr Val Arg Ser Val Tyr Asn
                85                  90                  95

Cys Trp Tyr Phe Gly Asp Ser Tyr Cys Lys Phe His Thr Cys Phe Asp
            100                 105                 110

Thr Ser Phe Cys Phe Ala Ser Leu Phe His Leu Cys Arg Ile Ser Val
        115                 120                 125

Asp Arg Tyr Ile Ala Val Thr Asp Pro Leu Thr Tyr Pro Thr Lys Phe
    130                 135                 140

Thr Val Ser Ile Ser Gly Ile Cys Ile Val Leu Ser Trp Phe Phe Ser
145                 150                 155                 160

Val Thr Tyr Ser Phe Ser Ile Phe Tyr Thr Gly Ala Asn Glu Glu Gly
                165                 170                 175

Ile Glu Glu Leu Val Val Ala Leu Thr Cys Val Gly Gly Cys Gln Ala
            180                 185                 190

Pro Leu Asn Gln Asn Trp Val Leu Leu Cys Phe Leu Leu Phe Phe Ile
        195                 200                 205

Pro Thr Val Ala Met Val Phe Ile Tyr Ser Lys Ile Phe Leu Val Ala
    210                 215                 220

Lys His Gln Ala Arg Lys Ile Glu Ser Thr Ala Ser Gln Ala Gln Ser
225                 230                 235                 240

Ser Ser Glu Ser Tyr Lys Glu Arg Val Ala Lys Arg Lys Arg Lys Ala
                245                 250                 255

Ala Lys Thr Leu Gly Ile Ala Met Ala Ala Phe Leu Val Ser Trp Leu
            260                 265                 270

Pro Tyr Ile Val Asp Ala Val Ile Asp Ala Tyr Met Asn Phe Ile Thr
        275                 280                 285

Pro Pro Tyr Val Tyr Glu Ile Leu Val Trp Cys Val Tyr Tyr Asn Ser

290                 295                 300

Ala Met Asn Pro Leu Ile Tyr Ala Phe Phe Tyr Pro Trp Phe Arg Lys
305                 310                 315                 320

Ala Ile Lys Leu Ile Val Ser Gly Lys Val Leu Arg Ser Asp Ser Ser
                325                 330                 335

Thr Thr Asn Leu Phe Ser Glu Glu Val Glu Lys Asp Gln
            340                 345

<210>  23
<211>  18
<212>  DNA
<213>  Pan troglodytes

<220>
<221>  schTAR1_5_01
<222>  (1)..(18)
<223>  5' primer

<400>  23
atgatgccct tttgccac                                                    18

<210>  24
<211>  27
<212>  DNA
<213>  Pan troglodytes

<220>
<221>  schTAR1_3_01
<222>  (1)..(27)
<223>  3' primer

<400>  24
ctatgaactc aattccaaaa ataattt                                          27

<210>  25
<211>  25
<212>  DNA
<213>  Pan troglodytes

<220>
<221>  schGPR58_5_01
<222>  (1)..(25)
<223>  5' primer

<400>  25
gaaacattca attgctctga atatg                                            25

<210>  26
<211>  21
<212>  DNA
<213>  Homo sapiens

<220>
<221>  schGPR58_3_02
<222>  (1)..(21)
<223>  3' primer

<400>  26
tgcttcaatt tattcatgca g                                                21

<210>  27
<211>  21
<212>  DNA
<213>  Pan troglodytes

<220>
<221>  chGPR58_ex1_5_02
<222>  (1)..(20)
<223>  5' primer

<400>  27
ctaaggagcc tgatctcaac c                                                21

<210>  28
<211>  20
<212>  DNA
<213>  Pan troglodytes

<220>
<221>  chGPR58_ex1_3_01
<222>  (1)..(20)
<223>  3' primer

<400>  28
gctctgtgtg atctccgttg                                                  20

<210>  29
<211>  19
<212>  DNA
<213>  Homo sapiens

<220>
<221>  schGPR57_5_02
<222>  (1)..(19)
<223>  5' primer

<400>  29
cagtgactca tcctcctgg                                                   19

<210>  30
<211>  21
<212>  DNA
<213>  Homo sapiens

<220>
<221>  schGPR57_3_02
<222>  (1)..(21)
<223>  3' primer

<400>  30
tctattcact tttgcaacag c                                                21

<210>  31
<211>  20
<212>  DNA
<213>  Pan troglodytes

<220>
<221>  schTAR2_5_01
<222>  (1)..(20)
<223>  5' primer

<400>  31
atgatgaatt tgcctgaccc                                                    20


<210>  32
<211>  21
<212>  DNA
<213>  Pan troglodytes


<220>
<221>  schTAR2_3_01
<222>  (1)..(21)
<223>  3' primer

<400>  32
ctaagcatgg gcagaaaaca g                                                  21


<210>  33
<211>  22
<212>  DNA
<213>  Pan troglodytes


<220>
<221>  schPNR_5_01
<222>  (1)..(22)
<223>  5' primer

<400>  33
atgagagctg tcttcatcca ag                                                 22


<210>  34
<211>  23
<212>  DNA
<213>  Pan troglodytes


<220>
<221>  schPNR_3_01
<222>  (1)..(23)
<223>  3' primer

<400>  34
tcattcttgg tacaaatcaa cag                                                23


<210>  35
<211>  24
<212>  DNA
<213>  Pan troglodytes


<220>
<221>  schTAR4_5_02
<222>  (1)..(24)
<223>  5' primer

<400>  35
cttctccata tgtaaataac agcg                                               24

<210>  36
<211>  25
<212>  DNA
<213>  Pan troglodytes

<220>
<221>  schTAR4_3_02
<222>  (1)..(25)
<223>  3' primer

<400>  36
gtatcctgaa cttcgtctat acaac                                          25

<210>  37
<211>  22
<212>  DNA
<213>  Homo sapiens

<220>
<221>  schTAR5_5_01
<222>  (1)..(22)
<223>  5' primer

<400>  37
atgaccagca atttttccta ac                                             22

<210>  38
<211>  26
<212>  DNA
<213>  Homo sapiens

<220>
<221>  schTAR5_3_01
<222>  (1)..(26)
<223>  3' primer

<400>  38
ttattctgaa aataaactaa tggctg                                         26

<210>  39
<211>  19
<212>  DNA
<213>  Pan troglodytes

<220>
<221>  schTAR3_5_01
<222>  (1)..(19)
<223>  5' primer

<400>  39
atggtgaaca atttctccc                                                 19

<210>  40
<211>  27
<212>  DNA
<213>  Pan troglodytes

<220>
<221>  schTAR3_3_01
<222>  (1)..(27)
<223>  3' primer

<400>  40
ttaatctttc tctacttctt cagaaaa                                          27

## Description

The present invention provides a fingerprint sequence which is specific and selective for chimp trace amine associated receptors (TAAR) forming a subfamily of G protein coupled receptors. The invention also provides the polypeptides identified as members of this family, nucleic acids encoding said polypeptides, and vectors, host cells and non-human animals comprising the novel family members. In addition, the invention provides methods of identifying TAARs.

Trace amines (TA) are endogenous compounds structurally related to biogenic amines and are found in the mammalian nervous system in trace amounts. TAs are stored in nerve terminals and released together with classical biogenic amines. To date there is no evidence for the existence of synapses using TAs as their exclusive transmitter. Recently, receptors specifically binding trace amines were reported by Borowski et al (Trace amines: identification of a family of mammalian G protein-coupled receptors. Proc Natl Acad Sci USA (2001) 98(16):8966-71) and Bunzow et al (Amphetamine, 3,4-methylenedioxymethamphetamine, lysergic acid diethylamide, and metabolites of the catecholamine neurotransmitters are agonists of a rat trace amine receptor. Mol Pharmacol. 2001, 60(6):1181-8.). These receptors apparently represent a novel GPCR-family.

The dysregulation of trace amines were linked to various psychiatric disorders like depression (Sandler M. et al., Decreased cerebrospinal fluid concentration of free phenylacetic acid in depressive illness. Clin Chim Acta. 1979, 93(1):169-71; Davis BA and Boulton AA. The trace amines and their acidic metabolites in depression--an overview. Prog Neuropsychopharmacol Biol Psychiatry. 1994, 18(1):17-45.), schizophrenia (Potkin SG et al., Phenylethylamine in paranoid chronic schizophrenia. Science. 1979, 206(4417):470-1; Sandler M and Reynolds GP. Does phenylethylamine cause schizophrenia? Lancet. 1976, 1(7950):70-1.) and bipolar disorder (Boulton AA.: Some aspects of basic psychopharmacology: the trace amines. Prog Neuropsychopharmacol Biol Psychiatry. 1982;6(4-6):563-70; Sabelli HC et al., Clinical studies on the KM/20.05.2005 phenylethylamine hypothesis of affective disorder: urine and blood phenylacetic acid and phenylalanine dietary supplements. J Clin Psychiatry. 1986 Feb; 47(2):66-70.). There has also been made a link between dysregulation of trace amines and attention-deficit hyperactivity disorder (Baker et al., Phenylethylaminergic mechanisms in attention-deficit disorder. Biol Phychiatry. 1991, 29(1):15-22), Parkinson's disease (Heller B and Fischer E., Diminution of phenethylamine in the urine of Parkinson patients. Arzneimittelforschung. 1973, 23(6):884-6.), migraine (D'Andrea G. et al., Elusive amines and primary headaches: historical background and prospectives. Neurol Sci. 2003, 24 Suppl 2:S65-7; D'Andrea, G. et al., Elevated levels of circulating trace amines in primary headaches. Neurology. 2004, 62(10):1701-1705.) and eating disorders (Wolf ME and Mosnaim AD. Phenylethylamine in neuropsychiatric disorders. Gen Pharmacol. 1983, 14(4):385-90; Branchek TA and Blackburn TP. Trace amine receptors as targets for novel therapeutics: legend myth and tact. Curr. Opin Pharmacol. 2003. 3(1):90-97.) such as i.e. obesity and anorexia as suggested by the very high structural similarity between PEA and amphetamine, which is considered the strongest anorexic compound known to date. (Samanin R, and Garattini S.: Neurochemical mechanism of action of anorectic drugs. Pharmacol Toxicol. 1993 Aug;73(2):63-8; Popplewell DA et al., A behavioural and pharmacological examination of phenylethylamine-induced anorexia and hyperactivity-comparisons with amphetamine. Pharmacol Biochem Behav. 1986 Oct;25(4):711-6.).

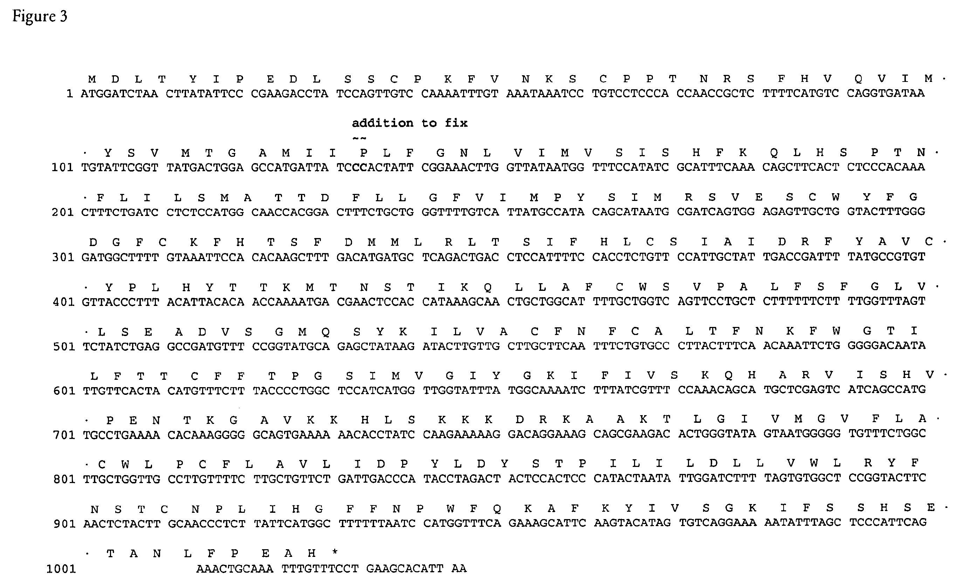

Therefore, there is a broad interest to increase the knowledge about trace amine receptors, especially to identify further trace amine receptors. However, examination of the literature and public database entries revealed inconsistencies in the naming of the TA receptors, e.g. the human receptor GPR102 (Lee et al., Discovery and mapping of ten novel G protein-coupled receptor genes. Gene. 2001, 275(1):83-91.) is also referred to as TA5 (Borowski et al Trace amines: identification of a family of mammalian G protein-coupled receptors. Proc Natl Acad Sci USA (2001) 98(16):8966-71)), and human 5-HT4Ψ (Liu et al., A serotonin-4 receptor-like pseudogene in humans. Brain Res Mol Brain Res. 1998, 53(1-2):98-103.) has also been named TA2Ψ (Borowski et al., Trace amines: identification of a family of mammalian G protein-coupled receptors. Proc Natl Acad Sci USA (2001) 98(16):8966-71). In addition, GPR57 (Lee et al., Cloning and characterization of additional members of the G protein-coupled receptor family. Biochim Biophys Acta. 2000, 1490(3):311-23.), GPR58 (Lee et al., Cloning and characterization of additional members of the G protein-coupled receptor family. Biochim Biophys Acta. 2000, 1490(3):311-23.) and PNR (Zeng et al., Cloning of a putative human neurotransmitter receptor expressed in skeletal muscle and brain. Biochem Biophys Res Commun. 1998, 242(3):575-8.) were so far not generally recognized as TA receptor. This inconsistencies cause confusion and uncertainty. Therefore, there is a strong need for a clear definition of this receptor family. In order to resolve any ambiguity of the current naming a novel, uniform nomenclature is proposed with this invention which refers to these receptors as Trace Amine Associated Receptors (TAARs). This novel nomenclature reflects the finding that at least some TAARs do not respond to TAs at all, hence the term "associated". It covers all receptors of this GPCR family and is compatible with the different number of receptor genes in different species. The novel nomenclature is strictly based on the sequential order of the receptor genes on the respective human chromosome as well as a detailed phylogenetic analysis (Fig. 3) of the receptor genes across different species. The nomenclature adheres to the following rules:
a) Any two (or three) genes that are orthologues, i.e. that were generated through a speciation event, should be labeled with the same number. Vice versa, two genes must not have the same number if they are not orthologous.
b) Genes that are paralogues, i.e. that were generated through a gene duplication event within the lineage of one species, should be distinguished by a letter suffix.
c) Examples: Genes hTAAR 5, rTAAR 5, mTAAR 5 are all orthologues.
   Genes mTAAR 8a, b, c in mouse are paralogues.
   Genes mTAAR 8a, b, c are all orthologues to hTAAR 8.

In addition, a comparison of the chimp TAAR proteins with the human equivalents may give indications to the functions of the human proteins. TAAR proteins, which are functional in both, human and chimpanzee, probably play an important physiologically role. On the other hand, TAAR proteins which are only functional in human but not in chimpanzee may account for the specific differences between human and chimpanzee.

The present invention provides novel chimp polypeptides identified as TAAR family members, the use of said polypeptides as drug target, the polynucleotide sequences encoding said polypeptide, and vectors, host cells and non-human animals comprising said polynucleotides. Furthermore, the present invention pertains the fingerprint motif specific and selective for the TAAR family and the use of it.

The present invention provides fingerprint motif comprising the sequence NSXXNPXXYXXXYXWF (SEQ. ID NO: 1), wherein X is any natural occurring amino acid. The term "fingerprint", "fingerprint motif" or "fingerprint sequence" as used herein relates to an amino acid sequence which is specific and selective for the chimp GPCR subfamily TAAR. Preferably, the fingerprint motif is specific for functional TAARs. The tryptophan residue in the context of the fingerprint motif is found exclusively in TAARs and not in any other known GPCR; rather, the corresponding sequence position is almost invariably occupied by polar or even charged amino acids in other GPCRs. The fingerprint motif may be used for identifying TAARs, preferably for identifying functional TAARs.

The present invention also provides a method of identifying chimp TAARs using the fingerprint sequence (SEQ. ID NO: 1). To be identified as a member of the chimp TAARs family a polypeptide may comprise a sequence having 100% identity with the fingerprint sequence.

Furthermore, the present invention pertains a method of identifying TAARs of other species, preferably of mammalians, using the fingerprint sequence (SEQ. ID NO: 1). To be identified as a member of the TAARs family a polypeptide may comprise a sequence having at least 75 % identity with the fingerprint sequence, preferably more than 87% identity, more preferably 100% identity.

The fingerprint sequence may be used as a "query sequence" to perform a search against sequence databases to, for example to identify TAAR family members. Such searches can be performed using a pattern recognition program as for example fuzzpro of EMBOSS (Rice et al., EMBOSS: the European Molecular Biology Open Software Suite. Trends in Genetics, 2000, 16(6):276-277). A search for chimp TAAR may be performed with fuzzpro, NSXXNPXXYXXXYXWF as the required sequence, number of mismatches = 0, database = swissprot (release 43).

The chimp TAAR family contains eight members (ptTAAR1 to ptTAAR 9) wherein five of them are pseudogenes: ptTAAR2 Ψ, ptTAAR3 Ψ, ptTAAR4 Ψ, ptTAAR8 Ψ, ptTAAR9 Ψ. All genes encoding the chimp TAAR family members are located on chimp chromosome 5. With the exception of ptTAAR 2, which is encoded by two exons, the coding sequences of the chimp TAAR genes are located in a single exon.

The term "gene" as used herein refers to any segment of DNA associated with a biological function. A gene is an ordered sequence of nucleotides located in a particular position on a particular chromosome that encodes a specific functional product.

The term "pseudogene" as used herein relates to an inactive gene which may have evolved by mutation events from an active ancestor gene. Inactive means that the gene is not translated to functional polypeptide.

The present invention provides an isolated or recombinant polypeptide comprising sequence SEQ. ID NO: 3. Said polypeptide was identified as trace amine associated receptor (TAAR) and was named ptTAAR1.

The term "polypeptide sequence" (e.g., a protein, polypeptide, peptide, etc.) as used herein relates to a polymer of amino acids comprising naturally occurring amino acids.

The term "isolated" means altered "by the hand of man" form the natural state. If an "isolated" composition or substance occurs in nature, it has been changed or removed from its original environment, or both. For example, a polynucleotide or a polypeptide naturally present in a living animal is not "isolated", but the same polynucleotide or polypeptide separated form the coexisting materials of its natural state is "isolated" as the term is employed herein.

The term "recombinant" when used with reference, e.g. to a polynucleotide or polypeptide typically indicates that the polynucleotide or polypeptide has been modified by the introduction of a heterologous (or foreign) nucleic acid of the alteration of a native nucleic acid, or that the protein or polypeptide has been modified by the introduction of a heterologous amino acid.

The present invention further pertains the polynucleotide sequence comprising SEQ. ID NO: 2 that encodes the polypeptide ptTAAR1.

The term "polynucleotide sequence" (e.g., a nucleic acid, polynucleotide, oligonucleotide, etc.) as used herein relates to a polymer of nucleotides comprising nucleotides A, C, T, U, G.

The present invention provides further an isolated or recombinant polypeptide comprising sequence SEQ. ID NO: 14. Said polypeptide was identified as trace amine associated receptor and was named ptTAAR5.

The present invention also pertains the polynucleotide sequence comprising SEQ. ID NO: 13 that encodes the polypeptide ptTAAR5.

The present invention provides further an isolated or recombinant polypeptide comprising sequence SEQ. ID NO: 16. Said polypeptide was identified as trace amine associated receptor and was named ptTAAR6.

The present invention also pertains the polynucleotide sequence comprising SEQ. ID NO: 15 that encodes the polypeptide ptTAAR6.

The present invention also provides the nucleotide sequences of ptTAAR2Ψ (SEQ. ID NO: 4). Furthermore, the present invention provides the repaired nucleotide sequences of ptTAAR2Ψ (ptTAAR2Ψf, SEQ. ID NO: 5). This invention further provides the polynucleotide sequences encoded by the fixed nucleotide sequence ptTAAR2Ψf (SEQ. ID NO: 6). The term "repaired gene" or "fixed gene" as used herein relates to a pseudogene whose sequence was altered in a way that the gene becomes active. The polynucleotide sequence of i.e. hTAAR3 is inactive due to a mutation that caused an early stop codon. The sequence was repaired by insertion of two nucleotides at position 133-134 (see Figure 3).

The present invention also provides the nucleotide sequences of ptTAAR3Ψ (SEQ. ID NO: 7). Furthermore, the present invention provides the repaired nucleotide sequences of ptTAAR3Ψ (ptTAAR3Ψf, SEQ. ID NO: 8). This invention further provides the polynucleotide sequences encoded by the fixed nucleotide sequence ptTAAR3Ψf (SEQ. ID NO: 9).

The present invention also provides the nucleotide sequences of ptTAAR4Ψ (SEQ. ID NO: 10). Furthermore, the present invention provides the repaired nucleotide sequences of ptTAAR4Ψ (ptTAAR4Ψf, SEQ. ID NO: 11). This invention further provides the polynucleotide sequences encoded by the fixed nucleotide sequence ptTAAR4Ψf (SEQ. ID NO: 12).

The present invention also provides the nucleotide sequences of ptTAAR8Ψ (SEQ. ID NO: 17). Furthermore, the present invention provides the repaired nucleotide sequences of ptTAAR8Ψ (ptTAAR8Ψf, SEQ. ID NO: 18). This invention further provides the polynucleotide sequences encoded by the fixed nucleotide sequence ptTAAR8Ψf (SEQ. ID NO: 19).

The present invention also provides the nucleotide sequences of ptTAAR9Ψ (SEQ. ID NO: 20). Furthermore, the present invention provides the repaired nucleotide sequences of ptTAAR9Ψ (ptTAAR9Ψf, SEQ. ID NO: 21). This invention further provides the polynucleotide sequences encoded by the fixed nucleotide sequence ptTAAR9Ψf (SEQ. ID NO: 22).

Furthermore, the present invention also provides isolated or recombinant chimp polypeptides comprising an amino acid sequence which comprise the fingerprint sequence (SEQ. ID NO: 1).

In a further aspect the invention relates to the use of the polypeptides of the invention as drug target. Preferably, the polypeptides of the invention are used as a drug target for identifying compounds useful in depression therapy, in schizophrenia therapy, in migraine therapy, or in therapy of attention-deficit hyperactivity disorder or eating disorder as anorexia or obesity. The drug target may be suitable for the design, screening and development of pharmaceutically active compounds.

One embodiment of the invention relates to the polypeptide having a polypeptide sequence SEQ. ID NO: 3, 6, 9, 12, 14, 16, 19 or 22 as drug target. Preferably, the polypeptides of the invention are used as a drug target for identifying compounds useful in depression therapy, in schizophrenia therapy, in migraine therapy, or in therapy of attention-deficit hyperactivity disorder or eating disorder as anorexia or obesity.

Another embodiment of the invention relates to chimp receptors having an amino acid sequence which comprise the fingerprint sequence (SEQ. ID. NO: 1) as drug target. Preferably, these receptors are drug target for identifying compounds useful in depression therapy, in schizophrenia therapy, in migraine therapy, or in therapy of attention-deficit hyperactivity disorder or eating disorder as anorexia or obesity.

The term "polypeptide of the invention" relates to the novel polypeptides provided in the present invention, i.e. ptTAAR1 to ptTAAR9.

The present invention also pertain vectors comprising polynucleotides of the invention, host cells which are transduced with said vectors and the production of the polypeptides of the invention by recombinant techniques. Preferably, the vector comprises the polynucleotide comprising SEQ. ID NO: 2, SEQ. ID NO: 5, SEQ. ID NO: 8, SEQ. ID NO: 11, SEQ. ID NO: 13, SEQ. ID NO: 15, SEQ. ID NO: 18 or SEQ. ID NO: 21 and preferably, the host cells are transduced with said vector.

Host cells can be genetically engineered (i.e., transduced, transformed or transfected) to incorporate expression systems or portion thereof for polynucleotides of the present invention. Introduction of the vector into host cells can be effected by methods described in many standard laboratory manuals, such as Davis et al., Basic methods in molecular biology Elsevier, New York (1986); Davis JM (ed.): Basic cell culture: a practical approach, sec. edition. Oxford University Press (2002); R. Ian Freshney: Culture of Animal Cells: A Manual of Basic Technique, fourth edition. John Wiley & Sons (Sd) 1999; and Sambrook et al., Molecular cloning: a laboratory manual, 2. Ed., Cold Spring Harbour Laboratory Press, Cold Spring Harbor, N.Y (1989), such as calcium phosphate transfection, DEAE-dextran mediated transfection, transvectin, microinjection, cationic lipid-mediated transfection, electroporation, transduction or infection.

A host cell may be mammalian cell as HEK 293, CHO, COS, HeLa, neuronal, neuroendocrinal, neuroblastomal or glial cell lines like SH-SY5Y, PC12, HN-10 (Lee HJ, Hammond DN, Large TH, Roback JD, Sim JA, Brown DA, Otten UH, Wainer BH.: Neuronal properties and trophic activities of immortalized hippocampal cells from embryonic and young adult mice. J Neurosci. 1990 Jun;10(6):1779-87.), IMR-32, NB41A3, Neuro-2a, TE 671, primary neuronal or glia cells from mammals like rat or mouse, Xenopus oocytes, bacterial cells such as streptococci, staphylocooci, *E*. *coli, Streptomyces* and *Bacillus subtilis* cells; fungal cells such as *Saccharomyces cerevisiae* and *Aspergillus* cell; insect cells such as *Drosophila* S2 and *Spodoptera* Sf9 cells and plant cells.

A great variety of expression systems can be used. Such a system include, among others, chromosomal, episomal and virus -derived systems, i.e. vectors derived from bacterial plasmids, from bacteriophage, from transposons, from yeast episoms, from yeast chromosomal elements, from viruses such as *baculovirus,* papova viruses, such as SV40, vaccinia viruses, adenovirus, fowl pox virus, pseudorabies, retroviruses and vectors derived from combinations thereof, such as those derived from plasmids and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth on Sambrook et al., Molecular cloning: a laboratory manual, 2. Ed., Cold Spring Harbour Laboratory Press, Cold Spring Harbour, N.Y. (1989) or Borowski et al Trace amines: identification of a family of mammalian G protein-coupled receptors. Proc Natl Acad Sci USA (2001) 98(16):8966-71).

The present invention further provides a transgenic non-human animal comprising a polynucleotide encoding a polypeptide of the invention. Preferably, the transgenic non-human animal comprises a polynucleotide comprising SEQ. ID NO: 2, SEQ. ID NO: 5, SEQ. ID NO: 8, SEQ. ID NO: 11, SEQ. ID NO: 13, SEQ. ID NO: 15, SEQ. ID NO: 18 or SEQ. ID NO: 21.

The transgenic non-human animal may be any non-human animal known in the art. Preferably, the transgenic non-human animal is a mammal, more preferably the transgenic non-human animal is a rodent. Most preferably, the transgenic animal of the invention is a mouse.

Methods of producing a transgenic non-human animal are well known in the art such as for example those set forth on Hogan, B.C., F; Lacy, E, *Manipulating the Mouse Embryo: A Laboratory Manual.* 1986, New York: Cold Spring Harbor Laboratory Press; Hogan, B., et al., Manipulating the mouse embryo. 1994, Second Edition, Cold Spring Harbor Press, Cold Spring Harbor and Joyner, A. (ed.): Gene Targeting - A Practical Approach Second Edition. Practical Approach Series, Oxford University Press 1999.

The polypeptides of the invention may be employed in a screening process for compounds which bind the receptor and which activate (agonists) or inhibit (antagonists) activation of the receptor polypeptide of the present invention or compounds which modulate receptor function e.g. by acting on receptor trafficking.

The present invention provides a method for identifying compounds which bind to a polypeptide of the invention comprising:
a) contacting a polypeptide of the invention with a candidate compound and
b) determining whether said compound binds to polypeptide of the invention.

The invention also provides a method for identifying compounds which have a stimulatory or inhibitory effect on the biological activity of polypeptide of the invention or its expression comprising
a) contacting a polypeptide of the invention with a candidate compound and
b) determining if said compound has modulated the function or activity of polypeptide of the invention.

In general, such screening procedures as described above involve producing appropriate cells which express the receptor polypeptide of the present invention on the surface thereof, however, the receptor expressed in appropriate cells may also be localized intracellularly. Such cells include i.e. cells from mammals, Xenopus, yeast, Drosophila or E. coli. Cells expressing the receptor (or cell membrane containing the expressed receptor) are then contacted with a test compound to measure binding, or stimulation or inhibition of a functional response.

One screening technique includes the use of cells which express receptor of this invention (for example, transfected CHO cells or HEK293) in a system which measures extracellular pH or intracellular calcium changes caused by receptor activation. In this technique, compounds may be contacted with cells expressing the receptor polypeptide of the present invention. A second messenger response, e.g., signal transduction, pH changes, PI hydrolyse, GTP-γ-[³⁵S] release or changes in calcium level, is then measured to determine whether the potential compound activates or inhibits the receptor.

Another method involves screening for receptor inhibitors by determining inhibition or stimulation of receptor-mediated cAMP and/or adenylate cyclase accumulation. Such a method involves transfecting a eukaryotic cell with the receptor of this invention to express the receptor on the cell surface. The cell is then exposed to potential antagonists in the presence of the receptor of this invention. The amount of cAMP accumulation is then measured. If the potential antagonist binds the receptor, and thus inhibits receptor binding, the levels of receptor-mediated cAMP, or adenylate cyclase, activity will be reduced or increased.

Another method for detecting agonists or antagonists for the receptor of the present invention is the yeast based technology as described in U.S. Patent 5,482,835.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

Examples of potential polypeptide of the invention antagonists include antibodies or, in some cases, oligonucleotides or proteins which are closely related to the ligand of the polypeptide of the invention, e.g., a fragment of the ligand, or small molecular weight molecules such as e.g. metabolites of neurotransmitters or of amino acids, which bind to the receptor but do not elicit a response, so that the activity of the receptor is prevented.

In addition, the present invention provides methods, polynucleotides, polypeptides, nucleic acid primer and uses substantially as herein before described especially with reference to the following examples.

Having now generally described this invention, the same will become better understood by reference to the specific examples, which are included herein for purpose of illustration only and are not intended to be limiting unless otherwise specified, in connection with the following figures.

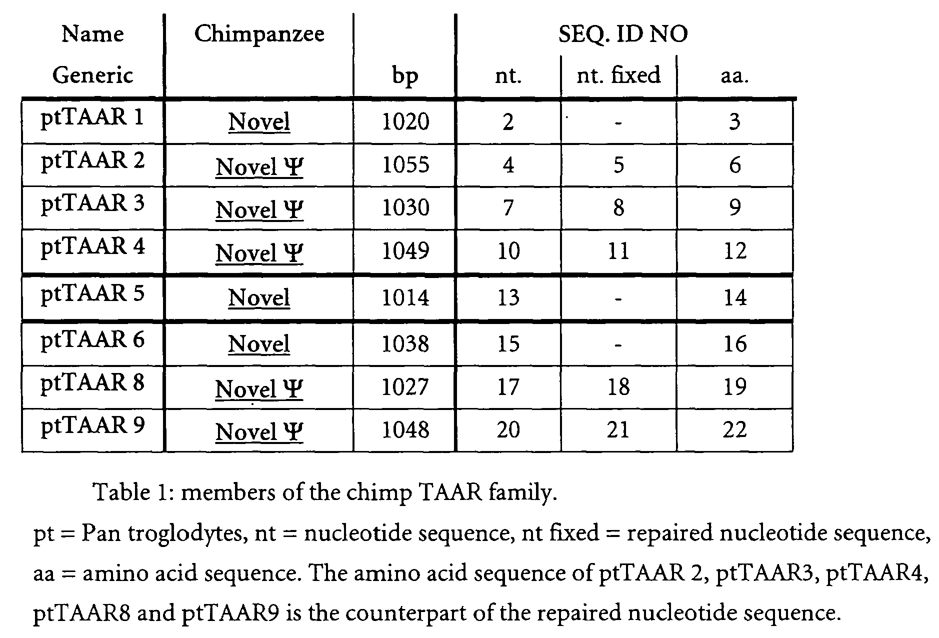

### Figures

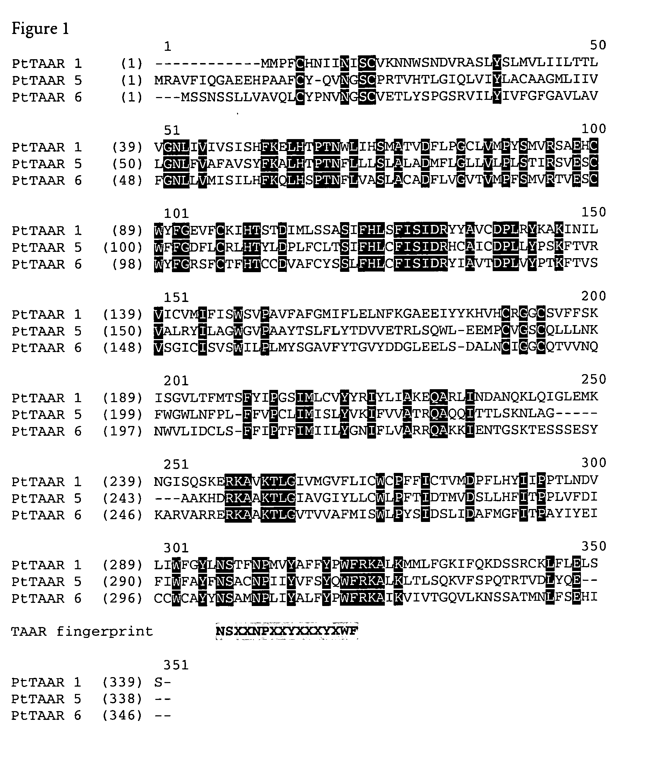

Figure 1 shows an alignment of all functional chimp TAARs. Amino acid residues conserved in all chimp, human, rat and mouse TAARs are highlighted by black shading. The characteristic chimp TAAR fingerprint motif is located in TM VII.

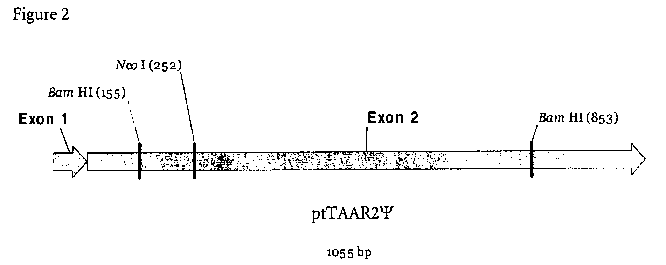

Figure 2 shows schematic pseudogene ptTAAR2Ψ. Restriction sites are given above the diagram.
Figure 3 shows the "repair" of the pseudogene hTAAR 3 (old name GPR57ψ): The two bps CC in position 133-134 of the fixed ORF were added, which repairs the otherwise early stop codon TCA.

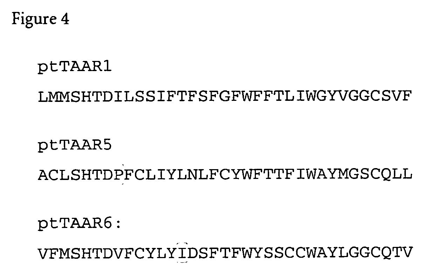

Figure 4 shows the ligand pocket vectors of the functional chimp TAARs. The differences to the human orthologues are indicated by grey boxes.
Figure 5 shows an alignment of human and chimp TAAR1.

### Examples

Commercially available reagents referred to in the examples were used according to manufacturer's instructions unless otherwise indicated.

### Identification of TAAR family members

The TAAR genes were identified by comparison of the previously published TAAR receptor genes with the chimp genomic sequence information available from Genbank employing standard algorithms like BLAST. Where the chimp genomic sequence information was not available, the information was extrapolated from the human genome sequences available from the Genbank.

Based on this sequence information the TAAR genes were amplified by means of PCR from chimp genomic DNA. Were not further specified, all procedures were carried out basically described in: Sambrook, J., Fritsch, E.F., und Maniatis, T. (1989). Molecular Cloning: A laboratory manual (New York: Cold Spring Harbor Laboratory Press). All reagents were of highest available purity, and all solutions and reagents were sterilized prior to use unless otherwise stated.

For this purpose oligonucleotide primers (table 2) were designed based on the TAAR coding sequences which had been derived from the genomic sequence information available at Genbank. The primers were designed such that the amplicons contain the entire open reading frames. The primer were designed using the VectorNTI software (Vector NTI version 9.0.0, Informax) following standard rules for PCR primers, mainly: **a**) Primers should have a length of 18-25 nt **b**) a G/C content of about 50% **c**) should not contain inverted repeats longer than 3 nt **d**) should carry an G or C at least at the 5' end **e**) should have no simple repetitions of the same nts more than 3, and **f**) the annealing temperatures (TM) of primers used in the same PCR reaction should differ as little as possible (for details see for example: McPherson M.J., Hames B.D., Taylor G.R. (eds.): PCR 2 - A Practical Approach. The practical approach series, Oxford University Press, 1995, ISBN: 0-19-963424-6). The annealing temperatures of the primer oligonucleotides were calculated based on rules described in: Breslauer KJ, Frank R, Blocker H, Marky LA.: Predicting DNA duplex stability from the base sequence. Proc Natl Acad Sci U S A. 1986 Jun;83(11):3746-50 (These rules assume that the sequences are not symmetric and contain at least one G or C and have a length of minimally 8 nts). Oligonucleotides were ordered from Microsynth (Microsynth AG, Schützenstrasse 15, 9436 Balgach, Switzerland) in HPLC purified quality.

Due to the draft quality of the chimpanzee genome sequence release the sequence of some ptTAAR genes, or parts thereof, were not available. Therefore, some primers had to be designed based on the corresponding human genome sequences. In those instances and wherever possible, primers where placed outside of the coding regions (table 2).

**Table 2: Primer for cloning of ptTAARs.**

| Receptor | Primer names | Sequence (5' → 3') | TM | SEQ. ID NO | Comment |
|---|---|---|---|---|---|
| ptTAAR 1 | schTAR1_5_01 | atgatgcccttttgccac | 59.2 | 23 | |
| | schTAR1_3_01 | ctatgaactcaattccaaaaaataattt | 57.4 | 24 | |
| ptTAAR 2 (exon II) | schGPR58_5_01 | gaaacattcaattgctctgaatatg | 58.9 | 25 | |
| | schGPR58_3_02 | tgcttcaatttattcatgcag | 56.3 | 26 | human |
| ptTAAR2 ( exon I) | chGPR58_ex1_5_02 | ctaaggagcctgatctcaacc | 57.8 | 27 | |
| | chGPR58_ex1_3_01 | gctctgtgtgatctccgttg | 59.2 | 28 | |
| ptTAAR 3 | schGPR57_5_02 | cagtgactcatcctcctgg | 56.1 | 29 | human |
| | schGPR57_3_02 | tctattcacttttgcaacagc | 55.4 | 30 | human |
| ptTAAR 4 | schTAR2_5_01 | atgatgaatttgcctgaccc | 59.5 | 31 | |
| | schTAR2_3_01 | ctaagcatgggcagaaaacag | 59.7 | 32 | |
| ptTAAR 5 | schPNR_5_01 | atgagagctgtcttcatccaag | 58.3 | 33 | |
| | schPNR_3_01 | tcattcttggtacaaatcaacag | 56.6 | 34 | |
| ptTAAR6 | schTAR4_5_02 | cttctccatatgtaaataacagcg | 57.0 | 35 | |
| | schTAR4_3_02 | gtatcctgaacttcgtctatacaac | 55.2 | 36 | |
| ptTAAR 8 | schTAR5_5_01 | atgaccagcaatttttcctaac | 57.1 | 37 | human |
| | schTAR5_3_01 | ttattctgaaaataaactaatggctg | 57.2 | 38 | human |
| ptTAAR 9 | schTAR3_5_01 | atggtgaacaatttctccc | 54.3 | 39 | |
| | schTAR3_3_01 | ttaatctttctctacttcttcagaaaa | 55.7 | 40 | |

| | | | | | |
|---|---|---|---|---|---|
| ptTAAR2: The two exons of this gene, predicted to encode GPR58 based upon the finding in the human gene, were cloned separately from genomic DNA; human: primers were designed based on the corresponding human genome sequences. (Tm: melting temperature in °C calculated according to Breslauer KJ, Frank R, Blocker H, Marky LA.: Predicting DNA duplex stability from the base sequence. Proc Natl Acad Sci U S A. 1986 Jun;83(11):3746-50; 5 in the primer name indicates a 5' primer (i.e. schTAR3_5_01) and 3 in the primer name indicates a 3' primer). | | | | | |

The actual PCR reactions had a total volume of 50 µl and the following composition: Template equaling 5-100 ng genomic DNA (source specified below), 200 nM oligonucleotide primer, 1.5 mM MgCl₂ (Invitrogen), 200 mM dNTPs each (Invitrogen), 1x concentrated PCR reaction buffer (Invitrogen) and 5 U/reaction recombinant Taq DNA polymerase (Invitrogen). The PCR reactions were assembled under a sterile working bench with UV irradiated equipment, and a) preparation of template material, b) assembly of PCR reactions, c) running the PCR reactions and agarose gel electrophoresis, and d) plasmid preparation were each performed in separated rooms in order to avoid any possible cross contamination of reagents with PCR amplified material or plasmid DNA. The PCR reactions were assembled in 200 µl PCR cups (Eppendorf) including Taq DNA polymerase at room temperature (RT) and transferred to the thermocycler (Geneamp 9700 with gold block, Applied Biosystems) preheated to 95°C. The temperature profile was adjusted as follows:

| | |
|---|---|
| 95°C | 2 min |

(95 °C: 30 sec, annealing temperature: 30 sec, 72°C: extension time) x cycle number:

| | |
|---|---|
| 72°C | 5 min |
| 4°C | max. 6 hrs |

**annealing temperature:** the Tm (melting temperature) of the oligonucleotide primer with the lower Tm value for the PCR reaction (calculated as specified above) -1°C was used as annealing temperature. Example: 2 primers with Tm primer 1 = 55°C, Tm primer 2 = 57°C ⇒ annealing temperature = 54°C.

**extension time** was calculated by length of amplicon (in kb) x 1 min.

**cycle number**: For each gene several PCR reactions were run in parallel with cycle numbers between 25 - 40. All PCR reactions were subsequently analyzed by agarose gel electrophoresis, and the PCR reaction with the lowest cycle number still producing a clearly visible PCR product of the correct size was used for subsequent cloning.

The PCR products were analyzed by agarose gel electrophoresis using 1% ultraPure agarose (GibcoBRL) made up in TAE buffer (Invitrogen). Agarose gels were run in PerfectBlue Horizontal Mini Electrophoresis Systems (Pequlab Biotechnologie GmbH, Erlangen, Germany) according to standard protocols (Sambrook et al., 1989). Agarose gels were stained with ethidium bromide, PCR products were visualized on a UV transilluminator (Syngene, Cambridge, UK), and the size of the PCR products was analyzed in comparison with the molecular weight standard 1 kb DNA ladder (Invitrogen). PCR products of the expected sizes were cut out from the gels with sterile scalpels (Bayha, Tuttlingen, Germany) and extracted from the agarose gel slices using the QIAquick Gel Extraction Kit (QIAGEN AG, Basel, Switzerland) following the instructions of the manufacturer.

The extracted PCR products were precipitated with a cold ethanol/sodium acetate precipitation (Sambrook et al., 1989), and DNA pellets were dissolved in a volume of 10 µl of 10 mM Tris/HCl pH 7.5. The PCR products in this solutions were subsequently cloned using the TOPO cloning kit for sequencing (Invitrogen; kits containing the vectors pCR4-TOPO and pCR2.1-TOPO were used) following the instructions of the manufacturer. Ligations were transformed into TOP10 chemically competent bacteria (included in the TOPO cloning kit for sequencing) following the instructions of the manufacturer and subsequently plated on LB agar plates containing 100 µg/ml ampicillin (Sigma, Division of FLUKA Chemie GmbH, Buchs, Switzerland) and incubated at 37°C over night. The bacterial colonies were analyzed by a method called "miniprep PCR": Colonies were picked with a sterile inoculation loop (Copan Diagnostic S.P.A., Brescia, Italy) and transferred to new LB agar plates containing 100 µg/ml ampicillin. The same inoculation loops were subsequently transferred individually into 50 µl 10 mM Tris/HCl pH 7.5 in 1.5 ml Eppendorf Cups (Eppendorf) in order to transfer the bacteria which had remained on the loop into the solution. This bacterial suspensions were subsequently heated to 95°C for 5 min, and 1 µl pf the resulting bacterial lysates per 50 µl PCR reaction was used as template for PCR reactions with primers flanking the multiple cloning site of the plasmids pCR4-TOPO and pCR2.1-TOPO (T7 and M13 reverse, sequences: Primer "T7": 5' - cgggatatcactcagcataat - 3', primer "M13 reverse": 5' - caggaaacagctatgacc - 3'). PCR reactions were assembled as described above and run with the following temperature profile:

| | |
|---|---|
| 95°C | 2 min |

(95 °C: 30 sec, 50°C: 30 sec, 72°C: 1 min) x 30 cycles

| | |
|---|---|
| 72°C | 5 min |
| 4°C | max. 6 hrs. |

The PCR products were analyzed by agarose gel electrophoresis as described above. Bacterial colonies for which cloned DNA fragments of the expected sizes could be detected by the method outlined above were used for subsequent plasmid preparation.

For plasmid preparations bacterial colonies identified to carry inserts of the expected sizes by "miniprep PCR" were used to inoculate bacterial liquid cultures in LB medium containing 100 µg/ml ampicillin, and cultures were incubated on a horizontal shaker at 37°C over night (Sambrook et al., 1989). Plasmids were isolated from the bacterial liquid cultures using the HiSpeed Plasmid Maxi Kit (QIAGEN AG, Basel, Switzerland) following the instructions of the manufacturer. Plasmid concentrations were determined by measuring the OD at 260 nm using an UV/VIS spectrophotometer ultrospec 3300 pro (Amersham Biosciences Europe GmbH, Otelfingen, Switzerland). The size of inserts in the isolated plasmids was analyzed by means of restriction digests using an restriction endonuclease i.e. EcoRI (New England Biolabs, products distributed in Switzerland by Bioconcept, Allschwil, Switzerland); EcoRI restriction sites are flanking the multiple cloning sites in both vectors pCR4-TOPO and pCR2.1-TOPO, therefore the cloned inserts can be released from the plasmids by EcoRI restriction digests. Of each plasmid 0.5 µg were digested with EcoRI in a total volume of 20 µl following the recommendations of the manufacturer. Restriction digests were analyzed by agarose gel electrophoresis as described above. Plasmids which turned out to carry inserts of the expected sizes as revealed by restriction analysis were subjected to DNA sequence analysis, carried out by an external company (Microsynth AG, Schützenstrasse 15, 9436 Balgach, Switzerland).

The DNA sequences of the cloned TAAR gene open reading frames, as revealed by DNA sequence analysis, were compared to either previously published sequences and/or to the sequence of the TAAR genes retrieved from the genome sequence information. Potential errors in the DNA sequence information which could have been introduced by the PCR reaction were eliminated by comparing the sequences of the two to three DNA plasmids which were cloned each from independent PCR reactions per TAAR gene: Since the probability of the same PCR error to occur at the very same position in independent PCR reactions is basically zero, the alignment of the independent sequences for each gene revealed the very correct DNA sequence.

Template material: chimpanzee (pan troglodytes) genomic DNA was purchased from Southwest National Primate Research Center (SNPRC, P.O. Box 760549, San Antonio, Texas, USA). The DNA concentration was determined by measuring the OD at 260 nm as described above and the DNA concentration was adjusted to 100 ng/µl. The DNA was stored in aliquots at 4°C.

### Cell culture

a) Subcloning: For expression of TAAR receptors in mammalian cell lines, DNA fragments carrying the entire TAAR open reading frames were subcloned from the pCR4-TOPO or pCR2.1-TOPO vectors (Invitrogen) to pIRES-NEO2 (Rees S et al., Bicistronic vector for the creation of stable mammalian cell lines that predisposes all antibioticresistant cells to express recombinant protein. Biotechniques. 1996 Jan;20(1):102-4, 106, 108-10).
   To this end, DNA fragments carrying the entire TAAR open reading frames were removed from the respective TOPO vectors by restriction digest with i.e. EcoRI and subsequent purification of the ~1kb fragments by agarose gel electrophoresis and gel extraction as described above. In parallel, the pIRES-NEO2 vector was linearized with EcoRI and dephosphorylated with shrimp alkaline phosphatase (Roche) following the instructions of the manufacturer. The DNA fragments carrying the entire TAAR open reading frames were ligated into the linearized pIRES-NEO2 vector using T4 DNA ligase (New England Biolabs) by mixing 30 ng of linearized pIRES-NEO2 vector, 100-300 ng of DNA fragments carrying the respective TAAR coding sequence, 1x ligation buffer (final concentration; provided with the enzyme as 10x concentrate) and 1 µl of T4 DNA ligase in a total volume of 20 µl. The reaction was proceeded at room temperature for 2-3 hrs, and the ligation was transformed into chemically competent TOP10 cells as described for the TOPO ligations. Plating of transformed bacteria and picking of bacterial clones was performed as described earlier for the PCR cloning of TAAR genes from genomic and cDNA. The picked bacterial clones were used to inoculate 5 ml liquid cultures in LB containing 100 µg/ml ampicillin. These liquid cultures were used for mini plasmid preparations using the QIAprep Miniprep Kit (QIAGEN), following the instructions of the manufacturer. The purified pIRES-NEO2 derived plasmid constructs were analyzed for the presence of inserts of the expected sizes by restriction analysis using EcoRI and agarose gel electrophoresis. Plasmids which were identified to carry inserts of the expected sizes were subjected to DNA sequence analysis carried out by Microsynth in order to make sure for the correct orientation and sequence of the inserts. Plasmids carrying the correct inserts in the correct orientation were used for expression of the respective TAARs in mammalian cell lines.
b) Cell culture: Basic cell culture handling and techniques used are described in Davis JM (ed.): Basic cell culture, sec. edition. Oxford University Press 2002, ISBN: 0199638535. TAARs were expressed in HEK cells (ATCC number: CRL-1573; described in Graham, F.L., Smiley, J., Russell, W.C., und Nairn, R. (1977). Characteristics of a human cell line transformed by DNA from human adenovirus type 5. Journal of General Virology 36, 59-74). The culture medium consisted of DMEM with Glutamax I, with sodium pyruvate, with pyridoxine, with 4500 mg/l glucose, Invitrogen Cat. # 31966-021; Penicillin/Streptomycin; fetal calf serum heat inactivated 10%. Cells were passaged at a ratio of 1:10 - 1:30 using Trypsin/EDTA (Invitrogen Cat. # 25300-062).

### Comparison of human and chimpanzee TAARs

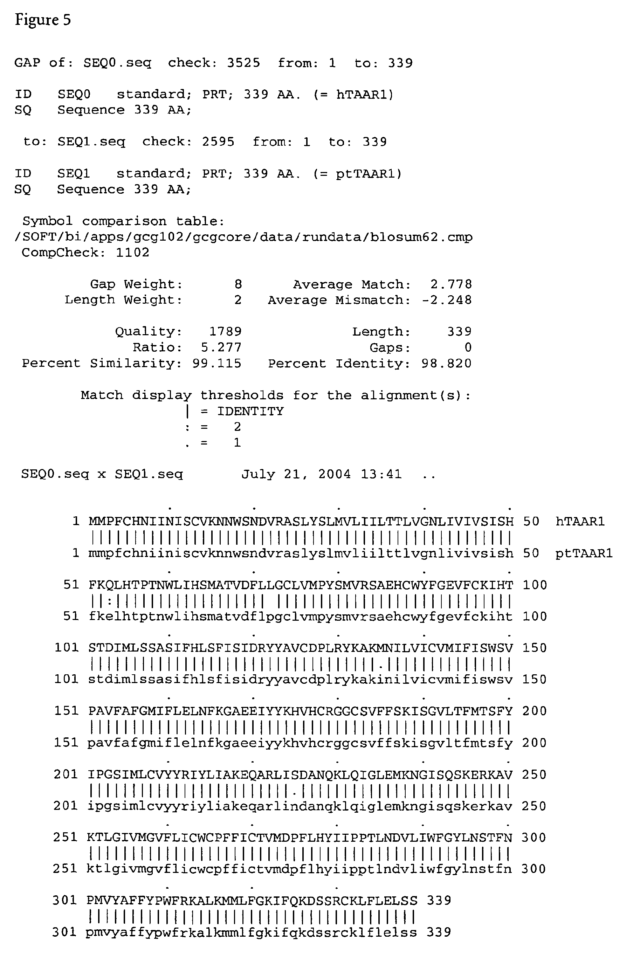

The average divergence between the nucleotide sequences of human and chimpanzee is just 1,2% (Clark et al., Science 2003, 302:1960-1963). The differences between the nucleotide sequences of the functional TAAR of human and chimpanzee is 0.9 to 1.3% (Table 3). The high grade of identity is also found between the amino acid sequences of the chimp TAAR proteins and the equivalent human TAAR proteins (Table 1, Figure 5). The pharmacology of the chimp TAAR is expected to be the same as of the human TAAR.

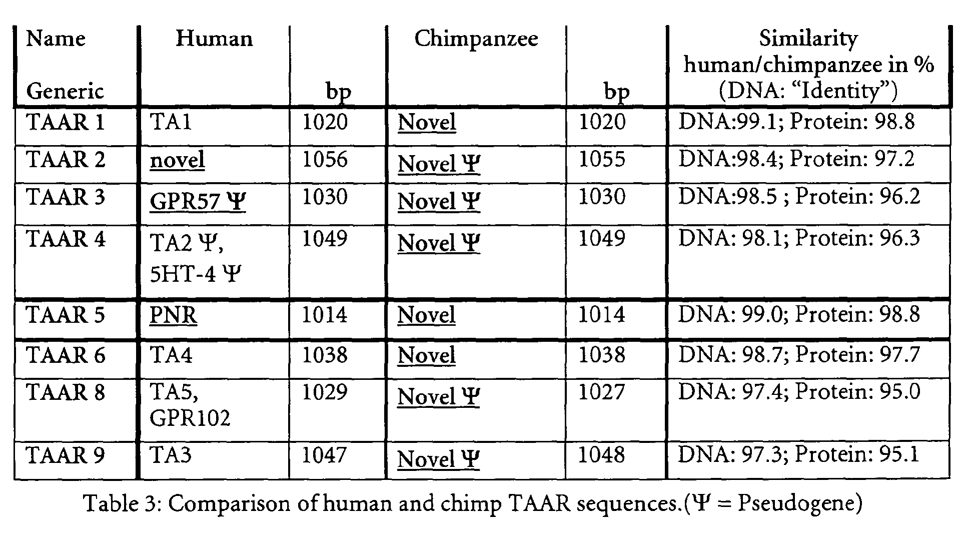

## Claims

1. An isolated or recombinant polypeptide comprising SEQ ID NO: 3.

2. An isolated or recombinant polypeptide comprising SEQ ID NO: 14.

3. An isolated or recombinant polypeptide comprising SEQ ID NO: 16.

4. Use of the polypeptide according any one of claims 1 to 3 as drug target.

5. Use of the polypeptide according claim 1 to 3 as drug target for identifying compounds useful in schizophrenia therapy.

6. Use of the polypeptide according claim 1 to 3 as drug target for identifying compounds useful in migraine therapy.

7. Use of the polypeptide according claim 1 to 3 as drug target for identifying compounds useful in depression therapy.

8. Use of the polypeptide according claim 1 to 3 as drug target for identifying compounds useful in therapy of eating disorders.

9. Use of the polypeptide according claim 1 to 3 as drug target for identifying compounds useful in therapy of attention deficit hyperactivity disorder.

10. An isolated or recombinant polynucleotide comprising SEQ. ID NO: 2.

11. An isolated or recombinant polynucleotide comprising SEQ. ID NO: 13.

12. An isolated or recombinant polynucleotide comprising SEQ. ID NO: 15.

13. A vector comprising the polynucleotide according to any one of claims 10 to 12.

14. A host cell comprising the vector according claim 13.

15. A transgenic non-human animal comprising the polynucleotide according to any one of the claims 10 to 12.

16. A chimp fingerprint motif comprising the sequence NSXXNPXXYXXXYXWF wherein X is any natural occurring amino acid.

17. Use of a sequence comprising the fingerprint motif according claim 16 for identifying chimp TAARs.

18. An isolated or recombinant chimp polypeptide having a sequence which comprises the fingerprint motif of claim 16.

19. A polypeptide according claim 18 as drug target.

20. A polypeptide according claim 18 as drug target for identifying compounds useful in schizophrenia therapy, in migraine therapy, in depression therapy, in therapy of eating disorder or in therapy of attention deficit hyperactivity disorder.

21. A method for identifying compounds which bind to the polypeptide according to any one of claim 1 to 3 or claim 18 comprising:
a) contacting the polypeptide according to any one of claim 1 to 3 or claim 18 with a candidate compound and
b) determining whether compound binds to said polypeptide.

22. A method for identifying compounds which have a stimulatory or inhibitory effect on the biological activity of a polypeptide according to any one of claim 1 to 3 or claim 18 comprising
a) contacting a polypeptide according to any one of claim 1 to 3 or claim 18 with a candidate compound and
b) determining if said compound has modulated the function or activity of said polypeptide.

23. Methods, polynucleotides, polypeptides, nucleic acid primer and uses substantially as herein before described especially with reference to the forgoing examples.
